# EUROPEAN PATENT APPLICATION

(11) **EP 4 613 774 A1**
(43) Date of publication of application: **10.09.2025**
(21) Application number: 23885084.6
(22) Date of filing: 03.11.2023
(51) Int. Cl.: C07K 16/30, C07K 19/00, C12N 15/13, C12N 15/62, C12N 15/63, C12N 5/10, C12N 5/0783, A61K 39/395, A61P 35/00

(54) **SINGLE DOMAIN ANTIBODY TARGETING HUMAN ROR1**

(30) Priority: 03.11.2022 CN 202211370878; 18.05.2023 CN 202310575171
(71) Applicant: Nanjing Probio Biotech Co., Ltd., Nanjing, Jiangsu 211100 (CN)
(72) Inventor: LI, Yingyu, Nanjing, Jiangsu 211100 (CN); JIA, Wenshuang, Nanjing, Jiangsu 211100 (CN); QI, Zeng, Nanjing, Jiangsu 211100 (CN); XIN, Yang, Nanjing, Jiangsu 211100 (CN); LI, Lianqu, Nanjing, Jiangsu 211100 (CN); ZHOU, Yi, Nanjing, Jiangsu 211100 (CN); CHEN, Li, Nanjing, Jiangsu 211100 (CN)
(74) Representative: Synergy IP Group AG
(86) International application number: PCT/CN2023/129542
(87) International publication number: WO 2024/094159

(57) **Abstract**

Provided in the present application are a single domain antibody targeting the ROR1 protein as well as a chimeric antigen receptor (CAR) and CAR-T cell comprising the single domain antibody. Also provided are uses of the single domain antibody and CAR-T cell in the treatment of tumors expressing the ROR1 protein.

## Description

### CROSS REFERENCE TO RELATED APPLICATIONS

The present application claims priority to Chinese patent application No. 202211370878.5, filed on November 3, 2022, and Chinese patent application No. 202310575171.6, filed on May 18, 2023, which are incorporated herein by reference in their entirety.

### TECHNICAL FIELD

The present disclosure belongs to the fields of tumor immunotherapy and molecular immunology, and in particular relates to the development of a more highly functional CAR-T targeting human ROR1 with high affinity, high specificity, and recognition of multiple antigen epitopes.

### BACKGROUND ART

Tumor immunotherapy has become one of the most important means of the treatment of tumors. Only after the cancer cells are identified, the immune system can effectively attack cancer cells. Carcinogenesis is the result of normal somatic cells losing their normal cellular regulatory functions and gene mutations accumulating to a certain extent. However, cancer cells can disguise themselves as normal somatic cells, dexterously avoiding the attack of the immune system.

A tumor-associated antigen (TAA) refers to an autoantigen expressed from tumor cells, which is partially present in normal host cells, is derived primarily from gene amplification or post-translational modification, but tends to be highly expressed or characteristically expressed in tumor cells. Many tumor-associated and tumor-specific antigens have been discovered at present, and the targeted therapy targeting TAAs is an important route to cancer treatment. There are already several cancer immunotherapeutic drugs on the market that rely on this mechanism, and these drugs are very effective clinically.

Monoclonal antibodies (mAbs) have become an increasingly important class of drugs, and their clinical application has revolutionized the field of cancer treatment. Different monoclonal antibodies have different antitumor mechanisms of action, including blocking tumor-specific growth factor receptors or immunomodulatory molecules, and complement- and cell-mediated tumor cell lysis. Rituximab targets CD20 antigen on B cells for the treatment of non-Hodgkin lymphoma, and trastuzumab (Herceptin) targets HER2 for the treatment of HER2-positive breast cancer, which are just two well-known examples. The development of drugs against TAAs remains a very active area of research. There are currently a large number of targeted drugs for different types of tumors that are being tested preclinically or clinically.

In general, antibody-mediated recognition of TAAs can dexterously direct NK cells or T cells to target corresponding TAA-highly expressing tumor cells. Antibodies against TAAs (e.g., rituximab and trastuzumab) can not only kill tumor cells directly by the ADCC effect, but also serve as diagnostic markers or innovatively increase the targeting of traditional cancer therapies.

Adoptive immunotherapy is a revolutionary treatment method in hematology, in which synthetic chimeric antigen receptors (CARs) are expressed in T cells through genetic engineering. CARs are engineered receptors that can redirect immune cells to target cancer cells. CAR-T cells have achieved impressive results in patients with hematological malignancies, and the FDA has approved 6 types of CAR-T cells for the treatment of relapsed or refractory B cell malignancies: Abecma, Breyanzi, Carvykti, Kymriah, Tecartus, and Yescarta. The components of these CARs (an extracellular antigen binding domain, hinge and transmembrane regions, a costimulatory domain, and an activation domain) are systematically designed to optimize cell activation and increase cell persistence. Using a different domain or making minor modifications to the domain sequence will greatly alter the efficacy of CAR-T cells. Despite the success of CAR-T in the treatment of hematologic tumors so far, many patients developed CAR-T cell-related toxicity and relapsed from the CAR-T cell therapy, which may be associated with binding epitopes of CARs, suggesting the necessity of developing antibodies with multiple binding epitopes. Next generation CAR-T cells are designed to reduce toxicity and prevent relapse by refining antigen targeting, regulating assembly or activation of CAR components, preventing anti-CAR immunity, and/or protecting cells in response to their surroundings. While CAR-T cells approved by the FDA target two types of B cell antigens, CD19 and B cell maturation antigen (BCMA), others targeting B cell malignancies, other hematological cancers, and solid tumors are emerging rapidly. Especially for solid tumors, there is an urgent need to develop novel CART therapies to meet the unmet clinical treatment needs of patients.

Receptor tyrosine kinase-like orphan receptor 1 (ROR1) is an oncofetal protein. As an orphan receptor tyrosine kinase-like surface antigen, it is expressed by many tissues during embryogenesis and plays a role in the development of the embryo's bones, heart and lungs, and nervous system. ROR1 is predominantly expressed in embryo tissues; and its expression levels in adult tissues are limited, including parathyroid gland, pancreatic islets, and esophagus, stomach, and duodenum areas. ROR1 is expressed in many B cell malignancies and various cancer cell lines, including solid tumors such as chronic lymphocytic leukemia (CLL), mantle cell lymphoma (MCL), and ovarian cancer. ROR1 is expressed on the surface of CLL cells and is an accurate and reliable marker of minimal residual disease in CLL. Higher levels of ROR1 expression are associated with higher circulating leukemia cells and disease invasiveness. ROR1 expression is retained on CLL cells during and after treatment. Thus, ROR1 is an ideal drug target for cancer treatment (Balakrishnan et al., Analysis of ROR1 Protein Expression in Human Cancer and Normal Tissues, Clin Cancer Res (2017) 23 (12): 3061-3071; Aghebati-Maleki et al., Receptor tyrosine kinase-like orphan receptor 1 (ROR-1): An emerging target for diagnosis and therapy of chronic lymphocytic leukemia, Biomedicine & Pharmacotherapy, Volume 88, April 2017, Pages 814-822; and De Propris et al., ROR1 is an accurate and reliable marker of minimal residual disease in chronic lymphocytic leukaemia, British Journal of Haematology, Volume 190, Issue 6, September 2020, Pages e346-e349).

### SUMMARY OF THE INVENTION

In one aspect, provided herein is an antibody or antigen-binding fragment thereof targeting a ROR1 protein, wherein the antibody comprises a heavy chain variable region comprising HCDR1, HCDR2, and HCDR3 selected from one of the following combinations:
(1) an amino acid sequence of HCDR1 as set forth in SEQ ID NO: 2;
   an amino acid sequence of HCDR2 as set forth in SEQ ID NO: 3; and
   an amino acid sequence of HCDR3 as set forth in SEQ ID NO: 4;
(2) an amino acid sequence of HCDR1 as set forth in SEQ ID NO: 7;
   an amino acid sequence of HCDR2 as set forth in SEQ ID NO: 8; and
   an amino acid sequence of HCDR3 as set forth in SEQ ID NO: 9;
(3) an amino acid sequence of HCDR1 as set forth in SEQ ID NO: 12;
   an amino acid sequence of HCDR2 as set forth in SEQ ID NO: 13; and
   an amino acid sequence of HCDR3 as set forth in SEQ ID NO: 14;
(4) an amino acid sequence of HCDR1 as set forth in SEQ ID NO: 17;
   an amino acid sequence of HCDR2 as set forth in SEQ ID NO: 18; and
   an amino acid sequence of HCDR3 as set forth in SEQ ID NO: 19;
(5) an amino acid sequence of HCDR1 as set forth in SEQ ID NO: 22;
   an amino acid sequence of HCDR2 as set forth in SEQ ID NO: 23; and
   an amino acid sequence of HCDR3 as set forth in SEQ ID NO: 24;
(6) an amino acid sequence of HCDR1 as set forth in SEQ ID NO: 27;
   an amino acid sequence of HCDR2 as set forth in SEQ ID NO: 28; and
   an amino acid sequence of HCDR3 as set forth in SEQ ID NO: 29;
(7) an amino acid sequence of HCDR1 as set forth in SEQ ID NO: 32;
   an amino acid sequence of HCDR2 as set forth in SEQ ID NO: 33; and
   an amino acid sequence of HCDR3 as set forth in SEQ ID NO: 34; and
(8) an amino acid sequence of HCDR1 as set forth in SEQ ID NO: 37;
   an amino acid sequence of HCDR2 as set forth in SEQ ID NO: 38; and
   an amino acid sequence of HCDR3 as set forth in SEQ ID NO: 39;
(9) an amino acid sequence of HCDR1 as set forth in SEQ ID NO: 66;
   an amino acid sequence of HCDR2 as set forth in SEQ ID NO: 3; and
   an amino acid sequence of HCDR3 as set forth in SEQ ID NO: 67;
(10) an amino acid sequence of HCDR1 as set forth in SEQ ID NO: 66;
   an amino acid sequence of HCDR2 as set forth in SEQ ID NO: 3; and
   an amino acid sequence of HCDR3 as set forth in SEQ ID NO: 71;
(11) an amino acid sequence of HCDR1 as set forth in SEQ ID NO: 66;
   an amino acid sequence of HCDR2 as set forth in SEQ ID NO: 3; and
   an amino acid sequence of HCDR3 as set forth in SEQ ID NO: 4;
(12) an amino acid sequence of HCDR1 as set forth in SEQ ID NO: 75;
   an amino acid sequence of HCDR2 as set forth in SEQ ID NO: 76; and
   an amino acid sequence of HCDR3 as set forth in SEQ ID NO: 19;
(13) an amino acid sequence of HCDR1 as set forth in SEQ ID NO: 78;
   an amino acid sequence of HCDR2 as set forth in SEQ ID NO: 76; and
   an amino acid sequence of HCDR3 as set forth in SEQ ID NO: 19;
(14) an amino acid sequence of HCDR1 as set forth in SEQ ID NO: 80;
   an amino acid sequence of HCDR2 as set forth in SEQ ID NO: 76; and
   an amino acid sequence of HCDR3 as set forth in SEQ ID NO: 19;
(15) an amino acid sequence of HCDR1 as set forth in SEQ ID NO: 82;
   an amino acid sequence of HCDR2 as set forth in SEQ ID NO: 83; and
   an amino acid sequence of HCDR3 as set forth in SEQ ID NO: 19;
(16) an amino acid sequence of HCDR1 as set forth in SEQ ID NO: 85;
   an amino acid sequence of HCDR2 as set forth in SEQ ID NO: 76; and
   an amino acid sequence of HCDR3 as set forth in SEQ ID NO: 19;
(17) an amino acid sequence of HCDR1 as set forth in SEQ ID NO: 85;
   an amino acid sequence of HCDR2 as set forth in SEQ ID NO: 87; and
   an amino acid sequence of HCDR3 as set forth in SEQ ID NO: 19;
(18) an amino acid sequence of HCDR1 as set forth in SEQ ID NO: 89;
   an amino acid sequence of HCDR2 as set forth in SEQ ID NO: 90; and
   an amino acid sequence of HCDR3 as set forth in SEQ ID NO: 19;
      or
   the antibody is a variant of an antibody defined by the amino acid sequences of HCDR1, HCDR2, and HCDR3 in any one of (1) to (18), wherein the variant comprises at least 1 and no more than 10, 9, 8, 7, 6, 5, 4, 3, or 2 amino acid changes in total in the sequences of HCDR1, HCDR2, and HCDR3, in comparison to the antibody defined in any one of (1) to (18).

In some embodiments, an amino acid sequence of the heavy chain variable region is selected from any one of:
(1) a sequence as set forth in SEQ ID NO: 1 or a heavy chain variable region sequence having at least 90% sequence identity thereto;
(2) a sequence as set forth in SEQ ID NO: 6 or a heavy chain variable region sequence having at least 90% sequence identity thereto;
(3) a sequence as set forth in SEQ ID NO: 11 or a heavy chain variable region sequence having at least 90% sequence identity thereto;
(4) a sequence as set forth in SEQ ID NO: 16 or a heavy chain variable region sequence having at least 90% sequence identity thereto;
(5) a sequence as set forth in SEQ ID NO: 21 or a heavy chain variable region sequence having at least 90% sequence identity thereto;
(6) a sequence as set forth in SEQ ID NO: 26 or a heavy chain variable region sequence having at least 90% sequence identity thereto;
(7) a sequence as set forth in SEQ ID NO: 31 or a heavy chain variable region sequence having at least 90% sequence identity thereto;
(8) a sequence as set forth in SEQ ID NO: 36 or a heavy chain variable region sequence having at least 90% sequence identity thereto;
(9) a sequence as set forth in SEQ ID NO: 65 or a heavy chain variable region sequence having at least 90% sequence identity thereto;
(10) a sequence as set forth in SEQ ID NO: 68 or a heavy chain variable region sequence having at least 90% sequence identity thereto;
(11) a sequence as set forth in SEQ ID NO: 70 or a heavy chain variable region sequence having at least 90% sequence identity thereto;
(12) a sequence as set forth in SEQ ID NO: 81 or a heavy chain variable region sequence having at least 90% sequence identity thereto;
(13) a sequence as set forth in SEQ ID NO: 84 or a heavy chain variable region sequence having at least 90% sequence identity thereto;
(14) a sequence as set forth in SEQ ID NO: 86 or a heavy chain variable region sequence having at least 90% sequence identity thereto; and
(15) a sequence as set forth in SEQ ID NO: 88 or a heavy chain variable region sequence having at least 90% sequence identity thereto.

In some embodiments, the antibody is a single domain antibody.

In some embodiments, the antibody is a humanized antibody.

In some embodiments, an amino acid sequence of a heavy chain variable region of the humanized antibody is selected from any one of:
(1) a sequence as set forth in SEQ ID NO: 41 or a heavy chain variable region sequence having at least 90% sequence identity thereto;
(2) a sequence as set forth in SEQ ID NO: 43 or a heavy chain variable region sequence having at least 90% sequence identity thereto;
(3) a sequence as set forth in SEQ ID NO: 45 or a heavy chain variable region sequence having at least 90% sequence identity thereto;
(4) a sequence as set forth in SEQ ID NO: 72 or a heavy chain variable region sequence having at least 90% sequence identity thereto;
(5) a sequence as set forth in SEQ ID NO: 73 or a heavy chain variable region sequence having at least 90% sequence identity thereto;
(6) a sequence as set forth in SEQ ID NO: 74 or a heavy chain variable region sequence having at least 90% sequence identity thereto;
(7) a sequence as set forth in SEQ ID NO: 77 or a heavy chain variable region sequence having at least 90% sequence identity thereto; and
(8) a sequence as set forth in SEQ ID NO: 79 or a heavy chain variable region sequence having at least 90% sequence identity thereto.

In some embodiments, the antibody targets the Frizzled domain or the Kringle domain of the ROR1 protein.

In some embodiments, the antibody has an EC₅₀ value of no higher than 0.1 µg/mL for the binding to the ROR1 protein, or the Frizzled domain or Kringle domain thereof as determined by ELISA.

In some embodiments, the antibody has a KD value of no higher than 10⁻⁶ M, preferably no higher than 10⁻⁷ M, more preferably no higher than 10⁻⁸ M for the binding to the ROR1 protein, or the Frizzled domain or Kringle domain thereof as determined by surface plasmon resonance technology.

In some embodiments, the antibody also comprises an Fc fragment; preferably, the Fc fragment is derived from a human IgG, such as IgG1.

In another aspect, provided herein is a fusion protein comprising at least one antigen-binding functional portion, wherein the antigen-binding functional portion comprises the above antibody or antigen-binding fragment thereof.

In some embodiments, the fusion protein comprises at least two antigen-binding functional portions targeting the same or different antigen epitopes, respectively.

In some embodiments, one of the two antigen-binding functional portions targets the Frizzled domain of the ROR1 protein, and the other of the two antigen-binding functional portions targets other portions on the ROR1 protein other than the Frizzled domain; one of the two antigen-binding functional portions targets the Kringle domain of ROR1, and the other of the two antigen-binding functional portions targets other portions on the ROR1 protein other than the Kringle domain; or one of the two antigen-binding functional portions targets the Kringle domain of ROR1, and the other of the two antigen-binding functional portions targets the Frizzled domain of ROR1.

In some embodiments, the antigen-binding functional portions are linked by a peptide linker molecule.

In another aspect, provided herein is a chimeric antigen receptor targeting a ROR1 protein, wherein the chimeric antigen receptor comprises an extracellular antigen binding domain comprising the above antibody or antigen-binding fragment thereof, or the above fusion protein.

In some embodiments, the chimeric antigen receptor comprises an amino acid sequence as set forth in any one of SEQ ID NOs: 56-63.

In another aspect, provided herein is a nucleic acid molecule encoding the above antibody or antigen-binding fragment thereof, the above fusion protein, or the above chimeric antigen receptor.

In some embodiments, the nucleic acid molecule comprises a nucleotide sequence as set forth in any one of SEQ ID NOs: 5, 10, 15, 20, 25, 30, 35, 40, 42, 44, and 46.

In another aspect, provided herein is an expression vector comprising the above nucleic acid molecule.

In another aspect, provided herein is a host cell comprising the above expression vector or expressing the above antibody or antigen-binding fragment thereof, the above fusion protein, or the above chimeric antigen receptor.

In some embodiments, the host cell is an immune effector cell and expresses the above chimeric antigen receptor.

In some embodiments, the immune effector cell is a T cell or an NK cell.

In another aspect, provided herein is a pharmaceutical composition comprising: 1) the above antibody or antigen-binding fragment thereof, the above fusion protein, the above nucleic acid molecule, or the above host cell; and 2) a pharmaceutically acceptable carrier.

In another aspect, provided herein is a method for treating a disease comprising administering to a subject in need thereof an effective amount of the above antibody or antigen-binding fragment thereof, the above fusion protein, the above nucleic acid molecule, the above host cell, or the above pharmaceutical composition.

In some embodiments, the disease is a tumor expressing the ROR1 protein.

In some embodiments, the tumor is selected from chronic lymphocytic leukemia, mantle cell lymphoma, and ovarian cancer.

In another aspect, provided herein is a kit for detecting the ROR1 protein in a sample, wherein the kit comprises the above antibody or antigen-binding fragment thereof, or the above fusion protein.

In another aspect, provided herein is use of the above antibody or antigen-binding fragment thereof, the above fusion protein, the above nucleic acid molecule, or the above host cell in the manufacture of a medicament for the treatment of a tumor.

In some embodiments, the disease is a tumor expressing the ROR1 protein.

In some embodiments, the tumor is selected from chronic lymphocytic leukemia, mantle cell lymphoma, and ovarian cancer.

### BRIEF DESCRIPTION OF THE DRAWINGS

Figure 1 shows the results of the ELISA experiment of purified monoclonal antibodies and the recombinant human ROR1-His protein.
Figure 2 shows the results of the ELISA experiment of purified monoclonal antibodies and the recombinant human ROR1 Kringle-His protein.
Figure 3 shows the results of the ELISA experiment of purified monoclonal antibodies and the recombinant human ROR1-His protein.
Figure 4 shows the results of the FACS experiment for the binding of purified monoclonal antibodies to the cell lines expressing human and mouse ROR1.
Figure 5 shows the results of the affinity experiment of humanized antibodies and their corresponding human ROR1-related proteins.
Figure 6 shows the results of the FACS experiment for the binding of humanized antibodies to the cell lines expressing human and mouse ROR1.
Figure 7 shows the graph of the flow cytometry detection results of the human ROR1-engineered cell line CHO-K1/ROR1.
Figure 8 shows the structural schematic diagram of the CARs constructed in the present disclosure.
Figure 9 shows the graph of the flow cytometry detection of the proportion of T cells in PBMCs after 4 days of activation.
Figure 10 shows the evaluation of the activation effect of human ROR1-targeted CARs on Jurkat cells based on a reporter gene method.
Figure 11 shows the evaluation of the activation effect of human ROR1-targeted CARs on Jurkat cells based on the cytokine IL-2 secretion level.
Figure 12 shows the specific killing effect of human ROR1-targeted CAR-T on target cells CHO-K1/ROR1/Luc.
Figure 13 shows the detection of cytokine release levels after co-incubation of human ROR1-targeted CAR-T with target cells CHO-K1/ROR1/Luc.
Figure 14 shows the results of the affinity experiment of affinity-matured antibodies of the AHP15485-VHH4 series and their corresponding human ROR1-related proteins.
Figure 15 shows the results of the affinity experiment of affinity-matured antibodies of the AHP15662-VHH4 series and their corresponding human ROR1-related proteins.

### DETAILED DESCRIPTION OF EMBODIMENTS

Unless otherwise stated, all technical and scientific terms used herein have the meanings commonly understood by those of ordinary skill in the art.

The term "or" refers to an individual element of the listed optional elements, unless the context explicitly indicates otherwise. The term "and/or" refers to any one of, any two of, any three or more of, or all of the listed optional elements.

The term "about" generally refers to a variation within a range of 0.5% to 10% above or below a specified value, for example 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

The term "contain" or "comprise" refers to the inclusion of the stated elements, integers, or steps, but not the exclusion of any other elements, integers, or steps. When the term "contain" or "comprise" is used herein, unless otherwise indicated, the situations where the subject matter consists of the described elements, integers, or steps are also encompassed. For example, when reference is made to an antibody variable region "comprising" a particular sequence, it is also intended to encompass the antibody variable region that consists of that particular sequence.

ROR1 (receptor tyrosine kinase-like orphan receptor 1) is an evolutionarily conserved membrane protein that is widely expressed in embryonic development and in a variety of human cancers. In terms of molecular structure, ROR1 comprises an extracellular domain consisting of an Ig-like domain, a Frizzled domain, and a juxtamembrane Kringle domain; a transmembrane domain; and a cytoplasmic domain consisting of a tyrosine kinase-like domain, two serine/threonine-rich domains, and a proline-rich domain (PRD). Because of its expression pattern and function in tumor progression, ROR1 has become a promising target for tumor therapy. The ROR1 protein includes a human or mouse ROR1 protein.

The term "antibody" as used herein is used in its broadest sense and includes immunoglobulins or other types of molecules comprising one or more antigen binding domains that specifically bind to an antigen, and refers to a protein or polypeptide that exhibits binding specificity for a particular antigen. Specific examples of antibodies can include intact antibodies (e.g., classical four-chain antibody molecules), single-chain antibodies, single domain antibodies, multispecific antibodies, etc. A classical antibody molecule is typically a tetramer consisting of 2 identical heavy chains and 2 identical light chains linked to each other by disulfide bonds. The heavy and light chains are divided into variable regions (V) at amino terminus and constant regions (C) at carboxy terminus, based on conservative differences in amino acid sequences. Variable regions are used to recognize and bind to antigens, and constant regions (such as Fc fragments) are used to initiate downstream effects such as antibody-dependent cell-mediated cytotoxicity (ADCC). Within the variable regions of the heavy and light chains, there are three localized regions with a higher degree of variation in the composition and sequence of amino acids, respectively, these three regions are key positions for antibody-antigen binding, and are therefore also referred to as complementarity determining regions (CDRs). The amino acid sequences of the CDRs can be determined readily using art-recognized numbering schemes, e.g., Kabat, Chothia, IMGT, AbM, or Contact. The three complementarity determining regions of the heavy chain are called HCDR1, HCDR2, and HCDR3, respectively, and the three complementarity determining regions of the light chain are called LCDR1, LCDR2, and LCDR3, respectively. Each heavy chain variable region (VH) and light chain variable region (VL) may be composed of three CDR and four FR regions, which may be arranged from amino terminus to carboxy terminus in the following order: FR1, CDR1, FR2, CDR2, FR3, CDR3, and FR4. In a specific embodiment, the CDR sequences of the antibody described herein have been determined according to the Kabat numbering scheme.

An "antigen-binding fragment" of an antibody molecule refers to a polypeptide that comprises partial sequences (especially the CDR sequences) of the source antibody and that has the binding specificity of the source antibody. The antigen-binding fragment usually comprises at least the heavy chain variable region of the source antibody and has antigen binding capability. There are many forms of antigen-binding fragments, e.g., Fab, Fab', F(ab')₂, single-chain fragment variable (scFv), and single domain antibody (sdAb). Those skilled in the art will know how to obtain these antigen-binding fragments. For example, a classical antibody molecule can be digested by papain to obtain a Fab fragment, digested by pepsin to obtain F(ab')₂, and treated with a reducing agent to form a Fab' fragment by cleaving the disulfide bonds in the hinge region of the F(ab')₂. A "single-chain fragment variable (scFv)" is a peptide chain composed of the heavy and light chain variable regions of an antibody linked by a short peptide. By properly folding, the variable regions from the heavy and light chains interact through non-covalent bonds to form Fv segments, so that scFv can better retain its antigen affinity.

A "single domain antibody (sdAb)", or also known as "V_{H}H antibody" refers to an antibody molecule that has antigen binding capacity, and comprises the variable region of a heavy chain, but no light chain. Structurally, a single domain antibody can also be considered a fragment of a classical four-chain antibody molecule. Single domain antibodies were first discovered in camelids, and then researchers have found more single domain antibodies with antigen binding capability by antibody library (e.g., phage display library) screening. Single domain antibodies have some advantages over ordinary antibody molecules (e.g., classical antibody molecules), for example including, but not limited to: a smaller molecular weight, so as to be readily accessible to tissues or sites that are difficult to reach by ordinary antibody molecules when used in humans, or able to access antigen epitopes in proteins or polypeptides that are difficult to reach by ordinary antibody molecules; and improved stability, so as to be able to resist for example changes in temperature and pH, as well as the action of denaturants and proteases.

The term "fusion protein" refers to a protein molecule composed of at least two different peptide segments, which is artificially generated (e.g., by genetic engineering technology). These peptide segments do not exist in nature or in the same protein molecule. Examples of common fusion proteins comprising antibody fragments include multispecific antibodies, antibody-cytokine fusion proteins, antibody-cytotoxin fusion proteins (also known as immunotoxins), enzyme-labeled antibodies for immunodetection, chimeric antigen receptors (CARs), etc. In a specific example, the fusion protein is a multispecific antibody that comprises at least two single domain antibodies provided herein that can bind to different antigen epitopes on the ROR1 protein.

An "epitope", also known as "antigenic determinant", refers to the site on an antigen that binds to the corresponding antibody molecule. The epitope can be a linear epitope or a conformational epitope. A linear epitope is composed of continuous amino acid residues. A conformational epitope comprises discontinuous amino acid residues, which are spatially close to each other to form a particular conformation, e.g., amino acid residues in a polypeptide that are not adjacent in the primary sequence of the polypeptide, but are sufficiently close to each other in the tertiary or quaternary structure of the polypeptide to be recognized and bound by the corresponding antibody.

An "Fc fragment" refers to the stem region of a Y-shaped antibody molecule, i.e., a fragment crystallizable (Fc), comprising the second and third constant domains (CH2 and CH3 domains) of a heavy chain. The Fc region of an antibody can be obtained by hydrolyzing the antibody molecule by a proteolytic enzyme (such as papain). In some examples, the Fc region may comprise a hinge, CH2, and CH3. When the Fc region comprises a hinge, the hinge can mediate dimerization between two Fc-containing polypeptides. The Fc fragment can be derived from IgG, IgM, IgD, IgE, or IgA. In some examples, the Fc region is derived from IgG1, IgG2, IgG3, or IgG4. The "Fc fragment" also includes a variant Fc fragment from a native Fc fragment that has been modified but retain its effector functions. A "variant Fc fragment" comprises an amino acid sequence having at least one amino acid change in the amino acid sequence of a native Fc fragment. In some examples, the variant Fc fragment has at least one amino acid substitution as compared to the parent Fc fragment (the native Fc fragment), for example, about 1 to about 10 amino acids, and preferably about 1 to about 5 amino acids in the parent Fc fragment, are substituted. In some examples, the variant Fc fragment has at least about 80% sequence identity, at least about 90% sequence identity, at least about 95%, at least about 96%, at least about 97%, at least about 98%, or at least about 99% sequence identity to the parent Fc fragment. Effector functions of an "Fc fragment" can include Fc receptor binding, Clq binding and complement-dependent cytotoxicity (CDC), antibody-dependent cell-mediated cytotoxicity (ADCC), mediation of phagocytosis, etc.

When referring to amino acid or nucleotide sequences, the term "sequence identity" (also known as "Sequence Identity") refers to the degree of identity between two amino acid or nucleotide sequences (e.g., a query sequence and a reference sequence), generally expressed as a percentage. Generally, before calculating the percentage of identity between two amino acid or nucleotide sequences, sequence alignment is performed first and gaps (if any) are introduced. If amino acid residues or bases are the same at a certain alignment position in two sequences, the two sequences are believed to be identical or matched at this position; and if the amino acid residues or bases are different in the two sequences, they are believed to be non-identical or mismatched at this position. In some algorithms, the number of matched positions is divided by the total number of positions in the alignment window to obtain sequence identity. In other algorithms, the number and/or the length of gaps is also taken into account. For the purpose of the present disclosure, the publicly available alignment software BLAST (available at website: ncbi.nlm.nih.gov) can be employed to obtain the optimal sequence alignment and calculate the sequence identity between two amino acid or nucleotide sequences using default settings. In some embodiments, "at least 90% sequence identity" as described herein includes, but is not limited to: at least 95%, at least 98%, at least 99%, or even 100% sequence identity.

"Target", "be directed against", or "specifically bind to" with respect to an antibody or antigen-binding fragment thereof, means that a molecule (e.g., an antibody or antigen-binding fragment thereof) has a higher binding affinity for another molecule (such as an antigen) as compared to other molecules that coexist in the environment. One molecule can target, be directed against, or specifically bind to more than one molecule, for example, a bispecific antibody can have a higher binding affinity for two different antigens than other molecules. The binding affinity of an antibody for an antigen can be measured by measurements of some parameters, such as the EC₅₀ or KD value for the binding of the antibody to the antigen.

EC₅₀ (concentration for 50% of maximal effect) refers to the concentration that produces 50% of the maximal effect. When used to express the binding capacity of an antibody molecule to the corresponding antigen in an enzyme-linked immunosorbent assay (ELISA), it can refer to the concentration of the antibody molecule that produces half of the maximum detection signal (such as colorimetric or fluorescent intensity). The lower the EC₅₀ value, the greater the binding affinity for the antigen.

The KD value can also be used to measure the binding affinity between an antibody and its antigen. The KD value is the equilibrium dissociation constant between an antibody and its antigen, i.e., the ratio of k_{off}/kₒₙ. Therefore, the lower the KD value (the lower the concentration), the higher the affinity of the antibody.

The terms "polypeptide" and "protein" are used interchangeably and refer to a polymer of amino acid residues. Such polymers of amino acid residues may contain natural or unnatural amino acid residues and include, but are not limited to, peptides, oligopeptides, dimers, trimers, and multimers composed of amino acid residues. Full-length proteins and fragments thereof are covered by this definition. The term also includes post-expression modifications of the polypeptide, for example glycosylation, sialylation, acetylation, phosphorylation, and similar modifications. Furthermore, for the purpose of the present disclosure, a "polypeptide" refers to a protein which comprises modifications, such as deletions, additions, and substitutions (generally conservative in nature), to the native sequence, as long as the protein maintains the desired activity. These modifications may be intentional, such as those induced via site-directed mutagenesis; or may be accidental, such as those induced via mutations of the host which produces the protein or errors due to PCR amplification.

When referring to antibodies or antigen-binding fragments thereof, the term "variant" as used herein refers to a protein which is obtained by introducing one or more amino acid insertions, deletions, or substitutions on the basis of the parent antibody molecule, and which still retains at least part of the functions of the parent antibody molecule (especially the function of interest, for example its binding capability to the corresponding antigen). For example, a variant of an antibody molecule may retain at least 20%, 30%, 40%, 50%, 60%, 70%, 80%, or 90% of the binding capacity of its parent antibody molecule to an antigen, or even have a higher binding capacity than the parent antibody molecule. In some embodiments, a variant of an antibody molecule can retain at least 80%, 85%, 90%, 95%, or even 100% or more of the binding affinity of its parent antibody molecule for the antigen. For an antibody molecule or antigen-binding fragment thereof, its variant typically comprises amino acid changes present in the backbone sequence of the variable region and/or the constant region, but do not exclude one or a few amino acid changes in the CDR region sequence. Thus, those skilled in the art will also appreciate that, corresponding variants of the anti-ROR1 protein antibody molecule provided by the present disclosure can be obtained by substitution, deletion, or addition of a few amino acids based on the particular antibody sequences provided herein and verifying or screening the resulting product for binding capability to the corresponding antigen (the ROR1 protein) or biological activity, which variants are also included within the scope of the present disclosure.

A "chimeric antigen receptor (CAR)" herein refers to an engineered membrane protein receptor molecule that confers the desired specificity on immune effector cells, such as the binding capability to a particular tumor antigen. A chimeric antigen receptor is usually composed of an extracellular antigen binding domain, a transmembrane domain, and an intracellular signaling domain. In some cases, the antigen binding domain is a stretch of scFv sequence or a single domain antibody fragment that is responsible for recognizing and binding to a specific antigen. The intracellular signaling domain typically comprises an immunoreceptor tyrosine-based activation motif (ITAM), such as a signaling domain derived from a CD3zeta molecule, which is responsible for activating immune effector cells to produce a killing effect. In addition, the chimeric antigen receptor may also comprise a signal peptide at the amino terminus which is responsible for the intracellular localization of a neoprotein, as well as a hinge region between the antigen binding domain and the transmembrane domain. In addition to the signaling domain, the intracellular signaling domain may also comprise a costimulatory domain derived from, for example, 4-1BB or CD28 molecule.

A "CAR cell" herein refers to a cell that expresses a CAR molecule on the surface of the cell. In most cases, the cell is an immune cell, such as a T cell or an NK cell. Accordingly, a CAR-expressing T cell is referred to herein as a "CAR-T" or "CAR-T cell". In addition, when referring to CAR-T cells herein, it refers not only to cells that have been directly modified with CAR, but also refers to progeny cells produced by these cells after they proliferate in vitro or in vivo, unless otherwise stated.

The terms "nucleic acid molecule", "nucleic acid", and "polynucleotide" are used interchangeably herein and refer to a polymer of nucleotides. Such polymers of nucleotides may contain natural and/or unnatural nucleotides and include, but are not limited to, DNA, RNA, and PNA. A "nucleic acid sequence" refers to a linear sequence of nucleotides comprised in a nucleic acid molecule or polynucleotide.

The term "vector" refers to a nucleic acid molecule that can be engineered to contain a polynucleotide of interest (e.g., a coding sequence for the polypeptide of interest) or a nucleic acid molecule (e.g., a nucleic acid, a plasmid, or a virus) that can be replicated in a host cell. The vector may comprise one or more of the following components: an origin of replication, one or more regulatory sequences that regulate the expression of the polynucleotide of interest (such as a promoter and/or an enhancer) and/or one or more selectable marker genes (such as antibiotic resistance genes and genes that can be used in colorimetric analysis, such as the β-galactose gene). The term "expression vector" refers to a vector that is used to express a polypeptide of interest in a host cell.

A "host cell" refers to a cell that can be or has been a recipient of a vector or an isolated polynucleotide. The host cell can be a prokaryotic cell or a eukaryotic cell. Exemplary eukaryotic cells include mammalian cells, such as primate or non-primate cells; fungal cells, such as yeast; plant cells; and insect cells. Non-limiting exemplary mammalian cells include, but are not limited to, CHO cells, HEK-293 cells, BHK cells, or PER-C6 cells, and derived cells thereof, such as 293-6E, CHO-DG44, CHO-K1, CHO-S, and CHO-DS cells. In some embodiments, the single domain antibodies provided herein can be secreted and produced by mammalian cells. Host cells include progeny of a single host cell, and the progeny may not necessarily be completely identical (in morphology or in genomic DNA complement) to the original parent cell due to natural, accidental, or deliberate mutation. Host cells may be isolated cells or cell lines, and also include cells transfected in vivo with the nucleic acid molecule or expression vector provided herein. In a specific example, the host cell is a CAR cell, such as a CAR-T cell.

"Subject" includes animals, such as mammals, including, but not limited to, primates, rodents, simians, felines, canines, equines, bovines, porcines, ovines, caprines, mammalian laboratory animals, mammalian farm animals, mammalian sport animals, and mammalian pets. The subject may be male or female and may be any subject of appropriate age, including infant, juvenile, adolescent, adult, and geriatric subjects. In some examples, a subject refers to an individual in need of diagnosis or treatment for a disease or condition. In some examples, the subject receiving the diagnosis or treatment may be a patient who is suffering from, or at risk of developing, a condition associated with the diagnosis or treatment. In a particular example, the subject is a human, such as a human patient. The term is often used interchangeably with "patient", "detection object", "treatment object", etc.

A "pharmaceutically acceptable carrier" as used in reference to pharmaceutical compositions refers to materials such as solid or liquid diluents, fillers, antioxidants, and stabilizers with which can be safely administered, and which are suitable for administration to humans and/or animals without undue adverse side effects, while maintaining the activity of the drug or active agent contained therein.

When referring to the treatment of a disease, an "effective amount" refers to the amount of an active compound (such as an antibody) that is sufficient to elicit the biological or medical response desired by a clinician in a subject. The "effective amount" of an antibody provided herein when administered can be determined by those skilled in the art based on factors such as the route of administration, as well as the subject's weight, age, and condition. For example, a typical daily dose range may be 0.01 mg to 100 mg of active ingredient per kg of body weight. The methods of administration of the active compounds (such as antibodies and fusion proteins) or immune effector cells provided herein include, but are not limited to, injection, for example, via intravenous, intramuscular, intraarterial, subcutaneous, intraperitoneal routes, etc.

### Antibody or antigen-binding fragment thereof targeting a ROR1 protein

Provided herein is an antibody or antigen-binding fragment thereof that specifically binds to the ROR1 protein. The antibody or antigen-binding fragment thereof binds to the ROR1 protein (or the Frizzled domain or the Kringle domain thereof) with a relatively high binding affinity. For example, as illustrated in the Examples below, the binding capability of the antibody or antigen-binding fragment thereof to the ROR1 protein (or the Frizzled domain or the Kringle domain thereof) can be measured by an assay method such as enzyme-linked immunosorbent assay (ELISA) and surface plasmon resonance (SPR) technology. In addition, it can also be determined by other protein-protein interaction assay methods known in the art, such as biolayer interferometry (BLI) technology.

In some embodiments, the antibody is a single domain antibody. In some embodiments, the single domain antibody is obtained by screening an alpaca native library (a single domain antibody phage display library) with the ROR1 protein (or the Frizzled domain or the Kringle domain thereof). In other embodiments, after the antibody sequence is known, the single domain antibody is obtained through genetic engineering technology, for example, it is obtained through introducing an expression vector expressing the antibody or antigen-binding fragment thereof into a host cell and culturing the host cell.

Provided herein are heavy chain CDR sequences of an antibody targeting the ROR1 protein (or the Frizzled domain or the Kringle domain thereof), and these sequences are as set forth in SEQ ID NOs: 2-4, 7-9, 12-14, 17-19, 22-24, 27-29, 32-34, or 37-39, respectively.

Based on the CDR sequences provided herein, those skilled in the art can construct various polypeptide constructs (including antibodies or antigen-binding fragments thereof) with the binding capability to the ROR1 protein (or the Frizzled domain or the Kringle domain thereof), including the use of framework regions (FRs) and/or constant regions from different antibody molecules in combination with these CDR sequences. These framework regions comprise native framework region sequences from human antibodies or animal (such as mouse, rat, sheep, and camel) antibodies. These framework regions may also comprise framework region sequence variants resulting from changes in the native framework region sequences. Polypeptide constructs that specifically bind to the ROR1 protein (or the Frizzled domain or the Kringle domain thereof) can be easily obtained by combining the CDR sequences provided herein with different framework region sequences to form the heavy chain variable region, and detecting its binding capability to the ROR1 protein (or the Frizzled domain or the Kringle domain thereof).

In some embodiments, the heavy chain variable region of an antibody or antigen-binding fragment thereof provided herein targeting the ROR1 protein (or the Frizzled domain or the Kringle domain thereof) comprises an amino acid sequence that has at least 20%, 30%, 40%, 50%, 60%, 70%, 80%, 85%, 90%, 95%, 96%, 97%, 98%, or 99% sequence identity to a sequence as set forth in SEQ ID NO: 1, 6, 11, 16, 21, 26, 31, or 36.

In some embodiments, also provided herein are humanized antibodies of the above single domain antibodies. These humanized antibodies are essentially identical in the CDR sequences to the above single domain antibodies, except for replacement of the alpaca antibody framework regions with human antibody framework regions.

In some embodiments, the heavy chain variable region of a humanized antibody or antigen-binding fragment thereof provided herein targeting the ROR1 protein (or the Frizzled domain or the Kringle domain thereof) comprises an amino acid sequence that has at least 20%, 30%, 40%, 50%, 60%, 70%, 80%, 85%, 90%, 95%, 96%, 97%, 98%, or 99% sequence identity to a sequence as set forth in SEQ ID NO: 41, 43, or 45.

On the basis of the above single domain antibodies (including humanized single domain antibodies), affinity maturation mutations were also made, and some clones with improved affinity were screened out. Accordingly, in some embodiments, provided are affinity-matured antibodies, and their CDR sequences are as follows: (1) an amino acid sequence of HCDR1 as set forth in SEQ ID NO: 66, an amino acid sequence of HCDR2 as set forth in SEQ ID NO: 3, and an amino acid sequence of HCDR3 as set forth in SEQ ID NO: 67; (2) an amino acid sequence of HCDR1 as set forth in SEQ ID NO: 66, an amino acid sequence of HCDR2 as set forth in SEQ ID NO: 3, and an amino acid sequence of HCDR3 as set forth in SEQ ID NO: 69; (3) an amino acid sequence of HCDR1 as set forth in SEQ ID NO: 66, an amino acid sequence of HCDR2 as set forth in SEQ ID NO: 3, and an amino acid sequence of HCDR3 as set forth in SEQ ID NO: 71; (4) an amino acid sequence of HCDR1 as set forth in SEQ ID NO: 66, an amino acid sequence of HCDR2 as set forth in SEQ ID NO: 3, and an amino acid sequence of HCDR3 as set forth in SEQ ID NO: 4; (5) an amino acid sequence of HCDR1 as set forth in SEQ ID NO: 75, an amino acid sequence of HCDR2 as set forth in SEQ ID NO: 76, and an amino acid sequence of HCDR3 as set forth in SEQ ID NO: 19; (6) an amino acid sequence of HCDR1 as set forth in SEQ ID NO: 78, an amino acid sequence of HCDR2 as set forth in SEQ ID NO: 76, and an amino acid sequence of HCDR3 as set forth in SEQ ID NO: 19; (7) an amino acid sequence of HCDR1 as set forth in SEQ ID NO: 80, an amino acid sequence of HCDR2 as set forth in SEQ ID NO: 76, and an amino acid sequence of HCDR3 as set forth in SEQ ID NO: 19; (8) an amino acid sequence of HCDR1 as set forth in SEQ ID NO: 82, an amino acid sequence of HCDR2 as set forth in SEQ ID NO: 83, and an amino acid sequence of HCDR3 as set forth in SEQ ID NO: 19; (9) an amino acid sequence of HCDR1 as set forth in SEQ ID NO: 85, an amino acid sequence of HCDR2 as set forth in SEQ ID NO: 76, and an amino acid sequence of HCDR3 as set forth in SEQ ID NO: 19; (10) an amino acid sequence of HCDR1 as set forth in SEQ ID NO: 85, an amino acid sequence of HCDR2 as set forth in SEQ ID NO: 87, and an amino acid sequence of HCDR3 as set forth in SEQ ID NO: 19; or (11) an amino acid sequence of HCDR1 as set forth in SEQ ID NO: 89, an amino acid sequence of HCDR2 as set forth in SEQ ID NO: 90, and an amino acid sequence of HCDR3 as set forth in SEQ ID NO: 19.

In some embodiments, the heavy chain variable region of an affinity-matured antibody provided herein targeting the ROR1 protein (or the Frizzled domain or the Kringle domain thereof) comprises an amino acid sequence that has at least 20%, 30%, 40%, 50%, 60%, 70%, 80%, 85%, 90%, 95%, 96%, 97%, 98%, or 99% sequence identity to a sequence as set forth in SEQ ID NO: 65, 68, 70, 72, 73, 74, 77, 79, 81, 84, 86, or 88.

Those skilled in the art will appreciate that, corresponding variants of the antibody molecule provided herein targeting the ROR1 protein (or the Frizzled domain or the Kringle domain thereof) can be obtained by substitution, deletion, or addition of a few amino acids based on the particular sequences provided herein and verifying or screening the resulting product for binding capability to the ROR1 protein (or the Frizzled domain or the Kringle domain thereof) or biological activity, which variants are also included within the scope of the present disclosure. For example, an antibody molecule provided herein can have at least 1 and no more than 10, e.g., no more than 5, 4, 3, 2, or 1 amino acid changes over its full-length, or in the variable region sequences or the CDR sequences. For example, there may be at least 1 and no more than 10, e.g., no more than 5, 4, 3, 2, or 1 amino acid changes in the heavy chain variable region sequence as set forth in SEQ ID NO: 1, 6, 11, 16, 21, 26, 31, or 36, and there may also be a total of no more than 5, 4, 3, 2, or 1 amino acid changes in SEQ ID NOs: 2-4, 7-9, 12-14, 17-19, 22-24, 27-29, 32-34, or 37-39, or the CDR sequences of an affinity-matured antibody, or the antibody has any combination of the above modifications.

It is contemplated that the antibody or antigen-binding fragment thereof described herein may comprise a conservative amino acid substitution. A conservative amino acid substitution can generally be described as the substitution of one amino acid residue with another amino acid residue of a similar chemical structure, and has little or essentially no effect on the function, activity, or other biological properties of the polypeptide. Conservative amino acid substitutions are well known in the art. A conservative substitution may, for example, be the substitution of one amino acid in one of the following groups (a) to (e) with another amino acid within the same group: (a) small aliphatic nonpolar or weakly polar residues: Ala, Ser, Thr, Pro, and Gly; (b) polar negatively charged residues and their (uncharged) amides: Asp, Asn, Glu, and Gln; (c) polar positively charged residues: His, Arg, and Lys; (d) large aliphatic nonpolar residues: Met, Leu, Ile, Val, and Cys; and (e) aromatic residues: Phe, Tyr, and Trp.

In some embodiments, the antibody or antigen-binding fragment thereof provided herein can further comprise a post-translational modification. Examples of post-translational protein modifications include: phosphorylation, acetylation, methylation, ADP-ribosylation, ubiquitination, glycosylation, carbonylation, ubiquitination-like, biotinylation, or addition of a polypeptide side chain or a hydrophobic group. Thus, a modified soluble polypeptide may comprise a non-amino acid component, such as a lipoid, a polysaccharide, or a monosaccharide, and a phosphate. A preferred form of glycosylation is sialylation modification, in which one or more sialic acid groups are bound to a polypeptide. Sialic acid groups improve the solubility and the serum half-life of the protein, and also reduce the potential immunogenicity of the protein.

### Fusion protein

Provided herein is a fusion protein comprising at least one antibody or antigen-binding fragment thereof provided herein that specifically binds to the ROR1 protein (or the Frizzled domain or the Kringle domain thereof) and at least one other functional portion. Examples of the fusion protein include, but are not limited to, multispecific antibodies (such as bispecific antibodies) and chimeric antigen receptors (CARs).

In some embodiments, the antibody or antigen-binding fragment thereof can be linked to an Fc fragment to form a fusion protein. The Fc fragment may be located at the C-terminus and N-terminus of the antibody or antigen-binding fragment thereof. Preferably, the Fc fragment may be located at the C-terminus of the antibody or antigen-binding fragment thereof. A fusion protein formed by the antibody or antigen-binding fragment thereof with the Fc fragment has the ability to specifically bind to the ROR1 protein (or the Frizzled domain or the Kringle domain thereof), and also has the effector functions of the Fc fragment, such as mediation of complement-dependent cytotoxicity (CDC), antibody-dependent cell-mediated cytotoxicity (ADCC), and phagocytosis. In addition, fusion with the Fc fragment can increase the half-life of the antibody or antigen-binding fragment thereof in vivo, so as to increase the dosing interval when the antibody or antigen-binding fragment thereof is used as a therapeutic drug.

In some embodiments, the antibody or antigen-binding fragment thereof can be linked to a protein tag to form a fusion protein. Protein tags can include purification tags and detectable tags. Purification tags include, but are not limited to, His6 tag, Flag tag, MBP tag, GST tag, SUMO tag, etc. Detectable tags can be used to indicate the presence or content of the ROR1 protein (or the Frizzled domain or the Kringle domain thereof) in a sample, or to track the location information of the ROR1 protein (or the Frizzled domain or the Kringle domain thereof) within a subject or within a cell. Examples of detectable tags include various enzymes that can be used in immunodetection, such as horseradish peroxidase (HRP) and alkaline phosphatase (ALP); and fluorescent proteins, such as GFP. Due to the specific binding capability of the antibody or antigen-binding fragment thereof to the ROR1 protein (or the Frizzled domain or the Kringle domain thereof), the amount of the antibody or antigen-binding fragment thereof can be determined by the amount of the detectable tag linked to the antibody or antigen-binding fragment thereof, and the content of the ROR1 protein (or the Frizzled domain or the Kringle domain thereof) in the sample is then determined.

In some embodiments, the antibody or antigen-binding fragment thereof can be linked to a cytokine or therapeutic protein to form a fusion protein. In this case, by virtue of the specific binding capability of the antibody or antigen-binding fragment thereof to the ROR1 protein (or the Frizzled domain or the Kringle domain thereof), the cytokine or therapeutic protein can be purposefully delivered to specific tissues or cells (e.g., tumor tissues expressing the ROR1 protein (or the Frizzled domain or the Kringle domain thereof)), achieving the therapeutic effects of the cytokine or therapeutic protein.

In some embodiments, the fusion protein is a bispecific antibody, wherein one antigen binding portion targets the ROR1 protein (or the Frizzled domain or the Kringle domain thereof) (e.g., from a single domain antibody provided herein), and the other antigen binding portion can bind to a second antigen or protein other than the ROR1 protein (or the Frizzled domain or the Kringle domain thereof). In other embodiments, the fusion protein is a bispecific antibody, wherein one antigen binding portion targets the ROR1 protein (or the Frizzled domain or the Kringle domain thereof), and the other antigen binding portion also targets the ROR1 protein (or the Frizzled domain or the Kringle domain thereof) (both from a single domain antibody provided herein). However, these two antigen binding portions differ in the amino acid sequences (especially the CDR sequences) and bind to different antigen epitopes on the ROR1 protein (or the Frizzled domain or the Kringle domain thereof), respectively. In a specific embodiment, one antigen binding portion targets the Frizzled domain, and the other antigen binding portion targets other sites on the ROR1 protein other than the Frizzled domain. In another specific embodiment, one antigen binding portion targets the Kringle domain, and the other antigen binding portion targets other sites on the ROR1 protein other than the Kringle domain. In another specific embodiment, one antigen binding portion targets the Kringle domain, and the other antigen binding portion targets the Frizzled domain.

In some embodiments, two antigen binding portions of the bispecific antibody are linked in tandem by a peptide linker (or linker sequence). The peptide linker can be a naturally occurring linker, a synthetic linker, or a combination of both. Particularly suitable linker sequences primarily comprise amino acid residues selected from glycine (Gly), serine (Ser), alanine (Ala), and threonine (Thr). For example, the linker may contain at least 75% (calculated based on the total number of residues present in the peptide linker) (such as at least 80%, at least 85%, or at least 90%) of amino acid residues selected from Gly, Ser, Ala, and Thr. The linker can also consist of only Gly, Ser, Ala, and/or Thr residues. In some embodiments, the linker contains 1-25 glycine residues, 5-20 glycine residues, 5-15 glycine residues, or 8-12 glycine residues. In some aspects, a suitable peptide linker typically contains at least 50% glycine residues, such as at least 75% glycine residues. In some embodiments, the peptide linker comprises only glycine residues. In some examples, the peptide linker comprises only glycine and serine residues, for example in the form of (GS)ₙ, where n is an integer of 1-20, for example. A fusion protein formed by linking two antigen binding portions through a peptide linker can be used in the extracellular antigen binding structure of a chimeric antigen receptor to form a bispecific chimeric antigen receptor.

In some embodiments, the second antigen is a tumor-associated antigen (TAA) or a tumor microenvironment-associated antigen (TMEAA). In some embodiments, the second antigen is an immunomodulatory antigen, wherein the antigen is associated with enhancing or inhibiting a signaling pathway in an immune cell. In some embodiments, the second antigen is a T cell surface molecule, such as a component of a T cell receptor complex, e.g., CD3 (including γ, δ, ε, ζ, and η chains).

### Chimeric antigen receptor (CAR)

The CAR herein may comprise an extracellular antigen binding domain that specifically binds to the ROR1 protein (or the Frizzled domain or the Kringle domain thereof), a transmembrane domain, an intracellular costimulatory signaling domain, and an intracellular signaling domain.

In some embodiments, the extracellular antigen binding domain of the CAR can comprise a single domain antibody (or antigen-binding fragment thereof) provided herein. The single domain antibody (or antigen-binding fragment thereof) can be linked to the transmembrane domain through a hinge region, such as a CD8α hinge. The CAR can be used to transduce immune effector cells (e.g., T cells) and be expressed on the cell surface. Therefore, also provided herein are a T cell expressing the chimeric antigen receptor and use of the T cell and/or the CAR in the manufacture of a medicament for the treatment of ROR1 protein-related diseases.

In some embodiments, the extracellular antigen binding domain may comprise two or more single domain antibodies (or antigen-binding fragments thereof) provided herein targeting the ROR1 protein (or the Frizzled domain or the Kringle domain thereof). These single domain antibodies (or antigen-binding fragments thereof) are linked in tandem, either directly or through peptide linkers. Preferably, these single domain antibodies (or antigen-binding fragments thereof) target different antigen epitopes on the ROR1 protein, respectively. For example, one single domain antibody (or antigen-binding fragment thereof) targets the Frizzled domain, and the other single domain antibody (or antigen-binding fragment thereof) targets other sites on the ROR1 protein other than the Frizzled domain; one single domain antibody (or antigen-binding fragment thereof) targets the Kringle domain, and the other single domain antibody (or antigen-binding fragment thereof) targets other sites on the ROR1 protein other than the Kringle domain; or one single domain antibody (or antigen-binding fragment thereof) targets the Kringle domain, and the other single domain antibody (or antigen-binding fragment thereof) targets the Frizzled domain.

A CAR provided herein can comprise a transmembrane domain that can comprise a polypeptide from a protein selected from: the α, β, or ζ chain of the T cell receptor, CD28, CD3zeta, CD45, CD4, CD5, CD8α, CD9, CD16, CD22, CD33, CD37, CD64, CD80, CD86, CD134, CD137, and CD154. In a specific example, the transmembrane domain may comprise an amino acid sequence as set forth in SEQ ID NO: 51 or a functional variant thereof.

A CAR provided herein can comprise a costimulatory domain that can comprise a polypeptide from a protein selected from: CD28, 4-1BB, OX40, and ICOS. In a specific example, the costimulatory domain may comprise an amino acid sequence as set forth in SEQ ID NO: 52 or a functional variant thereof.

A CAR provided herein can comprise an intracellular signaling domain that can comprise a signaling domain from CD3zeta. In a specific example, the intracellular signaling domain may comprise an amino acid sequence as set forth in SEQ ID NO: 53 or a functional variant thereof.

A CAR provided herein can comprise a hinge region that can link the extracellular antigen binding domain that specifically binds to the ROR1 protein (or the Frizzled domain or the Kringle domain thereof) and the transmembrane domain. In a specific example, the hinge region may comprise an amino acid sequence as set forth in SEQ ID NO: 50 or a functional variant thereof.

A CAR provided herein can comprise a signal peptide that can, for example, be located at the N-terminus of an extracellular antigen binding domain that specifically binds to the ROR1 protein (or the Frizzled domain or the Kringle domain thereof). The signal peptide may comprise an amino acid sequence as set forth in SEQ ID NO: 48 or a functional variant thereof.

A CAR provided herein can also be linked to a cleavable peptide. In an embodiment, the cleavable peptide may comprise an amino acid sequence from a T2A peptide. In a specific example, the cleavable peptide may comprise an amino acid sequence as set forth in SEQ ID NO: 54 or a functional variant thereof.

In some embodiments, the CAR can also be linked to a tEGFR fragment by a cleavable peptide, and the tEGFR fragment can be used for signal detection (e.g., indicating CAR-positive cells), or used as a molecular switch for CAR-T cells. In a specific example, the tEGFR fragment comprises an amino acid sequence as set forth in SEQ ID NO: 55 or a functional variant thereof.

In some specific embodiments, the CAR comprises an amino acid sequence as set forth in any one of SEQ ID NOs: 56-63. It will be appreciated that the sequences listed also comprise signal peptide sequences and tEGFR fragments linked by cleavable peptides, and that these portions are not functional parts of the CAR, and do not exist as a fusion protein with the rest after expressed in T cells. Thus, in other specific embodiments, a CAR provided herein comprises the remaining sequence after removing the signal peptide and/or the tEGFR fragment linked by the cleavable peptide from the amino acid sequence as set forth in any one of SEQ ID NOs: 56-63.

### Nucleic acid, vector, cell, and pharmaceutical composition

Provided herein is an isolated nucleic acid molecule that can encode the antibody or antigen-binding fragment thereof, fusion protein, or CAR described above. In some embodiments, the isolated nucleic acid molecule may comprise a nucleic acid sequence as set forth in any one of SEQ ID NOs: 5, 10, 15, 20, 25, 30, 35, 40, 42, 44, and 46, or a functional variant thereof. The nucleic acid molecules can be produced or synthesized by the following methods: (i) amplification in vitro, for example, production by polymerase chain reaction (PCR) amplification, (ii) production by cloning and recombination, (iii) purification, for example, fractionation by restriction enzyme digestion and gel electrophoresis, or (iv) synthesis, for example, by chemical synthesis. In certain embodiments, the isolated nucleic acid is a nucleic acid molecule prepared by recombinant DNA technology.

Also provided herein is a vector that may comprise the above nucleic acid molecule. The vector may be selected from one or more of a plasmid, a retroviral vector, and a lentiviral vector. In some embodiments, a nucleic acid molecule encoding a CAR is placed on a lentiviral expression vector for the preparation of CAR-T cells. For example, the lentiviral vector may comprise a nucleic acid sequence as set forth in any one of SEQ ID NOs: 5, 10, 15, 20, 25, 30, 35, 40, 42, 44, and 46, or a functional variant thereof. A "functional variant" as used herein refers to a different nucleotide sequence that encodes the same amino acid sequence due to codon degeneracy. Furthermore, other genes may also be comprised in the vector, such as marker genes that allow the vector to be selected in an appropriate host cell and under appropriate conditions. Furthermore, the vector may also comprise expression control elements that allow the coding region to be correctly expressed in an appropriate host. Such control elements are well known to those skilled in the art and may include, for example, promoters, ribosome binding sites, enhancers, and other control elements that regulate gene transcription or mRNA translation. In another aspect, the present application provides an immune effector cell that may comprise the CAR, the nucleic acid molecule, or the vector of the present application. In the present application, the immune effector cell may be a mammalian cell. In the present application, the immune effector cell may be selected from a T lymphocyte and a natural killer (NK) cell.

Provided herein is a method for preparing immune effector cells, comprising introducing the vector(s) described herein into the immune effector cells. For example, the vector(s) provided herein can be introduced into the immune effector cells, such as T lymphocytes or natural killer (NK) cells. In certain embodiments, each type of cell or each cell may comprise a vector or a type of vector. In certain embodiments, each type of cell or each cell may comprise multiple (e.g., 2 or more) vectors or multiple types of (e.g., 2 or more types of) vectors. The introduction of the vector(s) into the immune effector cells can be carried out by methods known in the art. For example, the immune effector cells can be transfected with retroviral vectors, and the viral genome carrying the CAR molecule can be integrated into the host genome, ensuring the long term and stable expression of the gene of interest. For another example, using transposons, a plasmid carrying the CAR (transposon) and a plasmid carrying a transposase are introduced into the target cells. For a further example, the CAR molecule can be added to the genome by means of gene editing (e.g., CRISPR/Cas9). In the present application, the vector with the CAR molecule described in the present application can be introduced into the cells by methods known in the art, such as electroporation and lipofection.

In another aspect, provided herein is a pharmaceutical composition that may comprise the above antibody or antigen-binding fragment thereof, the above fusion protein, the above nucleic acid molecule, the above vector, or the above immune effector cell, and a pharmaceutically acceptable carrier or excipient. Pharmaceutically acceptable carriers or excipients may include buffer agents, antioxidants, preservatives, low molecular weight polypeptides, proteins, hydrophilic polymers, amino acids, saccharides, chelating agents, counterions, metal complexes, and/or nonionic surfactants, etc. In the present application, the pharmaceutical composition may be formulated for oral administration, intravenous administration (e.g., intravenous injection, I.V.), intramuscular administration (e.g., intramuscular injection, I.M.), in situ administration at the site of the tumor, inhalation, rectal administration, transdermal administration, or administration through subcutaneous depots.

### Pharmaceutical use

Provided herein is use of the above antibody or antigen-binding fragment thereof, the above fusion protein, the above nucleic acid molecule, the above vector, or the above immune effector cell in the manufacture of a medicament for the treatment of a disease or condition associated with the expression of ROR1. In some embodiments, the disease or condition associated with the expression of ROR1 may be a tumor or cancer.

Also provided herein is a method for treating a disease comprising administering to a subject the above antibody or antigen-binding fragment thereof, the above fusion protein, the above nucleic acid molecule, the above vector, or the above immune effector cell. The subject has or is suspected of having a disease or condition associated with the expression of ROR1.

In some embodiments, the disease or condition associated with the expression of ROR1 is a tumor or cancer.

In some embodiments, the tumor includes solid tumors such as chronic lymphocytic leukemia (CLL), mantle cell lymphoma (MCL), and ovarian cancer.

Tumor immunotherapy by targeting ROR1 is a very useful therapeutic approach. The inventors have developed novel human ROR1-targeted specific functional monoclonal nanobodies (single domain antibodies) that recognize multiple antigen epitopes, as well as related chimeric antigen receptor T cell therapies, which are used to inhibit the growth of ROR1-positive tumor cells and treat cancer diseases in which ROR1 is expressed by specifically binding to different ROR1 antigen epitopes to reduce the likelihood of the development of drug resistance.

The present disclosure relates to a functional antibody and a CAR-T molecule directed against the human ROR1 target. The embodiments of the present disclosure will be described in detail below in combination with the Examples. Unless otherwise stated, the technical and scientific terms used in the present disclosure have the same meanings as commonly understood by those of ordinary skill in the art to which the present disclosure belongs. Unless otherwise stated, the methods and materials in the Examples described below are all conventional products that can be purchased on the market. Those skilled in the art to which the present disclosure belongs will understand that the methods and materials described below are exemplary only and should not be considered as limiting the scope of the present disclosure.

### Example 1: Obtainment of human ROR1 single domain antibody-positive clonal molecules

### 1) Screening lead antibody molecules specifically targeting human ROR1 using an alpaca native library

The alpaca native library developed independently by Probio Biotech was subjected to three sounds of liquid or solid phase screening with the human ROR1-Biotin protein (fused with a biotin protein tag, Acrobiosystems, RO1-H821y, referring to Q01973-1 for the sequence), the human ROR1 Frizzled-His domain protein (amino acid sequence at positions 165-305, fused with a His protein tag, Acrobiosystems, RO1-H5222, referring to Q01973-1 for the sequence), and the human ROR1 Kringle-His domain protein (amino acid sequence at positions 308-395, fused with a His protein tag, Acrobiosystems, RO1-H5223, referring to Q01973-1 for the sequence), respectively, to obtain a phage library eluate.

### 2) Screening positive clones

The neutralized phage panning eluate was added to a prepared TG1 bacterial solution. After mixing evenly, the mixture was left to stand at 37°C for 45 minutes to allow the phages to infect the TG1 host bacteria. After sufficient infection, the bacterial solution was gradiently diluted, and then plated on an agar plate with the relevant resistance, and the plate was inverted and incubated overnight at 37°C. A 96-well sterile deep well plate filled with 0.5 ml of 2YT medium (containing 0.2% w/v of glucose and 0.1 mg/ml of ampicillin antibiotic) was prepared. Monoclonal colonies cultured in the plate were picked with a sterile pipette tip into the well plate, and the plate was incubated overnight (16-18 hours) at 37°C with shaking at 220 rpm. 0.05-0.1 ml of monoclonal bacterial solution cultured overnight was removed and transferred to a newly prepared sterile deep well plate (filled with 0.5 ml of 2YT medium containing ampicillin antibiotic at a final concentration of 0.1 mg/ml), and cultured until the OD value of approximately 0.6-0.8 (under the detection conditions of OD600) was achieved. Helper phages were added, and the mixture was shaken and mixed evenly, and then left to stand for 45 minutes at 37°C for infection. After that, the plate was refilled with 0.5 ml of 2YT medium (containing 0.1 mg/ml of ampicillin antibiotic and kanamycin antibiotic at a final concentration of 0.05 mg/ml after addition), and incubated overnight (16-18 hours) with shaking at 25°C and 220 rpm. The overnight expressed bacterial solution was centrifuged at 4000 rpm for 10 minutes to obtain the phage display expression supernatant for subsequent ELISA binding assay of the monoclones after panning with the antigen proteins, namely the human ROR1-His protein (fused with a His protein tag, Acrobiosystems, RO1-H522y), the human ROR1 Frizzled-His domain protein, and the human ROR1 Kringle-His domain protein, as well as FACS binding assay for cell antigens, so as to obtain positive single domain antibody clones.

ELISA binding assay: Indirect ELISA was used to assess the binding capability of phage display antibodies in the supernatant to human ROR1-related proteins. ELISA plates were coated with 1 µg/ml of recombinant human ROR1-His protein, human ROR1 Frizzled-His domain protein, and human ROR1 Kringle-His domain protein in 100 µl/well of CBS coating reagent overnight at 4°C. The plates were washed with PBS-T (0.05% Tween) and blocked with 300 µl/well of PBS containing 3% skim milk at 37°C for 1 hour. Subsequently, the blocking solution was discarded, 50 µl of phage expression supernatant and 50 µl of 0.1% PBST were added to each plate, and the plates were then incubated for 2 hours at room temperature. The plates were washed three times with PBST and incubated with 100 µl/well of horseradish peroxidase-conjugated goat anti-M13 phage antibody (Sino Biological, Inc.) for 45 minutes at room temperature. The plates were washed six times with PBST, a TMB chromogenic solution (GenScript) was then added, and the plates were incubated for 10-15 minutes at room temperature in the dark. The reaction was stopped by adding 50 µl of 1 M HCl stop solution (Sigma). The plates were read at 450 nm using a microplate reader.

FACS assay: A FACS binding experiment was used to assess the binding capability of antibodies in the supernatant to human ROR1 antigens expressed on the membrane surface of CHO cells. CHO cells expressing the human ROR1 and parental cells as the negative control for detection were collected and washed 3 times with PBS. 2.5 X 10⁵ test cells, 100 ul of supernatant to be tested, and 3.5 µg/ml of biotin-labeled anti-phage antibody were added to a 96-well plate, and the plate was incubated at 4°C for 1 hour. The cells were then washed 3 times with PBS, 100 µl of iFluor-labeled streptavidin protein (Jackson, 016-600-084) was added, and the plate was incubated at 4°C for 45 minutes. Finally, the cells were washed 3 times with PBS, and the signals were read using FACS BD Calibur.

### Example 2: Sequencing of variable regions of positive clones and recombinant production of monoclonal antibodies

Positive clones were selected based on the ELISA and FACS assay results, and the original colonies corresponding to the positive clones were pipetted for bacterial solution culture. The expression plasmids were extracted from the cultured bacterial solution, and subjected to PCR and monoclonal Sanger sequencing. Finally, 8 positive clones were obtained, as shown in Table 1 below.

**Table 1. Positive clones and their predicted binding sites**

| Clone name | Predicted binding site |
|---|---|
| AHP15485 | Ig-like domain or spatial site |
| AHP15547 | Frizzled domain |
| AHP15580 | Kringle domain |
| AHP15662 | Frizzled domain |
| AHP15768 | Ig-like domain or spatial site |
| AHP15773 | Ig-like domain or spatial site |
| AHP15776 | Frizzled domain |
| AHP16026 | Kringle domain |

The sequences of the antibodies are as shown below:
The amino acid sequence of the heavy chain variable region of the single domain antibody AHP15485: SEQ ID NO: 1
The amino acid sequence of the CDR1 region of AHP15485: SEQ ID NO: 2
The amino acid sequence of the CDR2 region of AHP15485: SEQ ID NO: 3
The amino acid sequence of the CDR3 region of AHP15485: SEQ ID NO: 4
The DNA sequence of the heavy chain variable region of the single domain antibody AHP15485: SEQ ID NO: 5
The amino acid sequence of the heavy chain variable region of the single domain antibody AHP15547: SEQ ID NO: 6
The amino acid sequence of the CDR1 region of AHP15547: SEQ ID NO: 7
The amino acid sequence of the CDR2 region of AHP15547: SEQ ID NO: 8
The amino acid sequence of the CDR3 region of AHP15547: SEQ ID NO: 9
The DNA sequence of the heavy chain variable region of the single domain antibody AHP15547: SEQ ID NO: 10
The amino acid sequence of the heavy chain variable region of the single domain antibody AHP15580: SEQ ID NO: 11
The amino acid sequence of the CDR1 region of AHP15580: SEQ ID NO: 12
The amino acid sequence of the CDR2 region of AHP15580: SEQ **ID** NO: 13
The amino acid sequence of the CDR3 region of AHP15580: SEQ ID NO: 14
The DNA sequence of the heavy chain variable region of the single domain antibody AHP15580: SEQ ID NO: 15
The amino acid sequence of the heavy chain variable region of the single domain antibody AHP15662: SEQ ID NO: 16
The amino acid sequence of the CDR1 region of AHP15662: SEQ ID NO: 17
The amino acid sequence of the CDR2 region of AHP15662: SEQ ID NO: 18
The amino acid sequence of the CDR3 region of AHP15662: SEQ ID NO: 19
The DNA sequence of the heavy chain variable region of the single domain antibody AHP15662: SEQ ID NO: 20
The amino acid sequence of the heavy chain variable region of the single domain antibody AHP15768: SEQ ID NO: 21
The amino acid sequence of the CDR1 region of AHP15768: SEQ ID NO: 22
The amino acid sequence of the CDR2 region of AHP15768: SEQ ID NO: 23
The amino acid sequence of the CDR3 region of AHP15768: SEQ ID NO: 24
The DNA sequence of the heavy chain variable region of the single domain antibody AHP15768: SEQ ID NO: 25
The amino acid sequence of the heavy chain variable region of the single domain antibody AHP15773: SEQ ID NO: 26
The amino acid sequence of the CDR1 region of AHP15773: SEQ ID NO: 27
The amino acid sequence of the CDR2 region of AHP15773: SEQ ID NO: 28
The amino acid sequence of the CDR3 region of AHP15773: SEQ ID NO: 29
The DNA sequence of the heavy chain variable region of the single domain antibody AHP15773: SEQ ID NO: 30
The amino acid sequence of the heavy chain variable region of the single domain antibody AHP15776: SEQ ID NO: 31
The amino acid sequence of the CDR1 region of AHP15776: SEQ ID NO: 32
The amino acid sequence of the CDR2 region of AHP15776: SEQ ID NO: 33
The amino acid sequence of the CDR3 region of AHP15776: SEQ ID NO: 34
The DNA sequence of the heavy chain variable region of the single domain antibody AHP15776: SEQ ID NO: 35
The amino acid sequence of the heavy chain variable region of the single domain antibody AHP16026: SEQ ID NO: 36
The amino acid sequence of the CDR1 region of AHP16026: SEQ ID NO: 37
The amino acid sequence of the CDR2 region of AHP16026: SEQ ID NO: 38
The amino acid sequence of the CDR3 region of AHP16026: SEQ ID NO: 39
The DNA sequence of the heavy chain variable region of the single domain antibody AHP16026: SEQ ID NO: 40

A DNA fragment containing the codon-optimized heavy chain variable region was synthesized and inserted into the pcDNA3.4-Fc (HulgG1) expression vector to form an expression plasmid.

After HEK293-6E cells were transfected with the above plasmid and cultured for 10 days in a shake flask at 37°C, the supernatant was harvested for antibody purification. Prior to purification, the pipelines and the Protein A column were depyrogenated with 0.2 M NaOH. The column was re-equilibrated with a buffer containing 0.05 M Tris and 1.5 M NaCl (pH = 8.0). Subsequently, the harvested cell culture supernatant was diluted at 1 : 1 with 2× the above buffer and sterilized by filtration. After the filtered supernatant and the protein A column were incubated for 2 hours at room temperature and the column was washed with 1× the above buffer, IgG was eluted with sterile 0.1 M sodium citrate (pH 3.5), and the eluate was collected and neutralized with one-ninth volume of sterile 1 M Tris-HCl (pH = 9.0). Under sterile conditions, the product buffer was exchanged to PBS (pH 7.4) to remove any elution buffer, and the sample was concentrated. After concentration, the antibodies were quantified by OD280 nm using an extinction coefficient Ec of 1.43 (0.1%).

The purified antibodies were analyzed by SDS-PAGE using 10% precast gel (GenScript) and a BioRad electrophoresis system. The gel was stained with Estain 2.0 (GenScript), and the molecular size and purity were estimated by comparing the stained band to Protein Ladder (GenScript).

### Example 3: ELISA binding assay of monoclonal antibodies to human ROR1 recombinant proteins

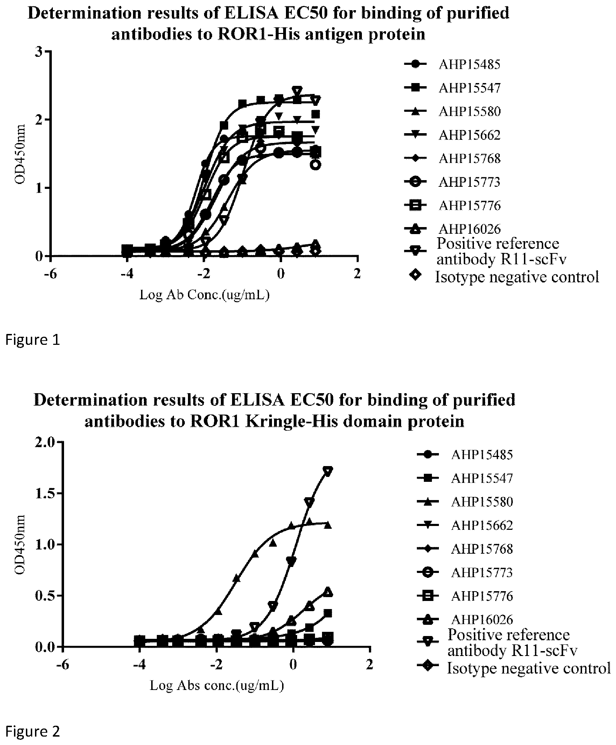

Indirect ELISA was used to assess the binding capability of the above purified antibodies to human ROR1-related recombinant proteins. ELISA plates (Nunc) were coated with 1 µg/ml of human ROR1-His protein, human ROR1 Frizzled-His domain protein, and human ROR1 Kringle-His domain protein in 100 µl/well of CBS overnight at 4°C. The plates were washed with PBS-T (0.05% Tween) and blocked with 300 µl/well of PBS containing 3% skim milk at 37°C for 1 hour. Subsequently, the blocking solution was discarded, and 100 µl of purified antibody at 8 µg/ml (about 100 nM) was added to the first well and diluted according to a 3-fold gradient to obtain a total of 11 test concentration gradients. The plates were then incubated for 1 hour at room temperature. The plates were washed 3 times with PBST and incubated with 100 µl/well of horseradish peroxidase-conjugated mouse anti-human IgG Fc fragment (GenScript) for 0.5 hours at 37°C. The plates were washed five times with PBST, a TMB chromogenic solution (GenScript) was then added, and the plates were incubated for 15 minutes at room temperature in the dark. The reaction was stopped by adding 50 µl of 1 M HCl stop solution (Sigma). The plates were read at 450 nm using a microplate reader. Figures 1-3 show the results of the ELISA experiment on the binding of 8 positive recombinant antibody clones to ROR1-related antigen proteins, and the EC₅₀ for each antibody is as shown in Table 2 below: the antigen binding capacity of the antibodies tested was all equal to or higher than that of the positive reference antibody R11-scFv.

**Table 2. Determination of ELISA EC₅₀ for binding of purified antibodies to ROR1-related proteins**

| Purified antibody | Human ROR1-His protein ELISA EC50 (ug/mL) | Human ROR1 Kringle-His protein ELISA EC50 (ug/mL) | Human ROR1 Frizzled-His protein ELISA EC50 (ug/mL) |
|---|---|---|---|
| AHP15485 | 0.006102 | NA | NA |
| AHP15547 | 0.01053 | NA | 0.01655 |
| AHP15580 | 0.04067 | 0.02941 | NA |
| AHP15662 | 0.01049 | NA | 0.01046 |
| AHP15768 | 0.02034 | NA | NA |
| AHP15773 | 0.01546 | NA | NA |
| AHP15776 | 0.01086 | NA | 0.02303 |
| AHP16026 | NA | 1.909 | NA |
| Positive reference antibody R11-scFv | 0.1045 | 1.167 | NA |
| Isotype negative control | NA | NA | NA |

### Example 4: Binding of monoclonal antibodies to cell lines expressing human and mouse ROR1

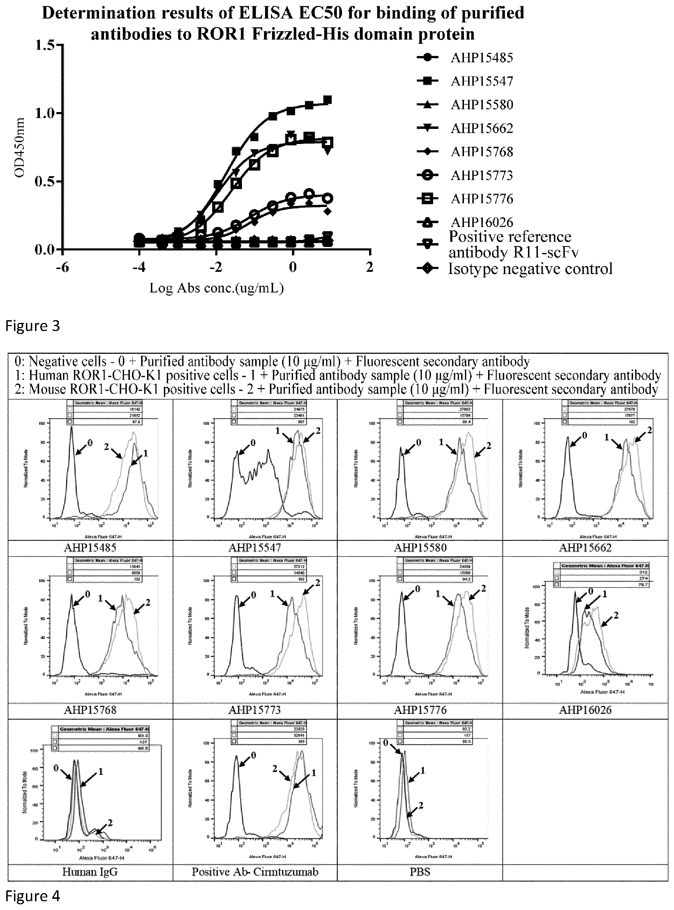

CHO cells expressing human and mouse ROR1 and parental cells as the negative control for detection were collected and washed 3 times with PBS. 2.5 X 10⁵ test cells and 100 ul of purified antibody at 10 µg/ml were added to a 96-well plate, and the plate was incubated at 4°C for 1 hour. The cells were then washed 3 times with PBS, 100 µl of iFluor-labeled goat anti-human IgG, an Fcy-specific fragment antibody, was added, and the plate was incubated at 4°C for 45 minutes. Finally, the cells were washed 3 times with PBS, and the signals were read using FACS BD Calibur. As shown in Figure 4, all the antibodies bound to the ROR1-expressing CHO-K1/Human ROR1 stable cells and CHO-K1/Mouse ROR1 stable cells but did not bind to the CHO parental cells.

### Example 5: Humanized design and recombinant production of human ROR1-targeted single domain antibody molecules

The parental antibody structure was modeled by the computer-aided homology modeling software (MOE). A human native germline sequence with high homology to the parental sequence was selected. The CDRs of the positive monoclonal antibody were grafted into the human native germline sequence using CDR grafting technology to obtain the humanized chimeric antibody of the parental antibody. By comparing the different amino acid residues between the chimeric antibody and the parental antibody, the key sites that might affect the subsequent affinity based on classic residues, interaction loop regions, the core region, mutation hotspots, etc. were identified, and appropriate sites for combinatorial design were selected to obtain the amino acid sequence of the variable region of the humanized antibody. The amino acid sequence of the humanized antibody was codon-optimized, and a DNA fragment containing the codon-optimized heavy chain variable region was synthesized and inserted into the pcDNA3.4-Fc (Human IgG1) expression vector to form an expression plasmid. The production of relevant expression plasmids was carried out according to the method of recombinant expression and purification of the antibody of Example 2 to obtain humanized antibodies.
The amino acid sequence of the heavy chain variable region of the single domain antibody AHP15485-VHH4: SEQ ID NO: 41
The amino acid sequence of the CDR1 region of AHP15485-VHH4: SEQ ID NO: 2
The amino acid sequence of the CDR2 region of AHP15485-VHH4: SEQ ID NO: 3
The amino acid sequence of the CDR3 region of AHP15485-VHH4: SEQ ID NO: 4
The DNA sequence of the heavy chain variable region of the single domain antibody AHP15485-VHH4: SEQ ID NO: 42
The amino acid sequence of the heavy chain variable region of the single domain antibody AHP15662-VHH4: SEQ ID NO: 43
The amino acid sequence of the CDR1 region of AHP15662-VHH4: SEQ ID NO: 17
The amino acid sequence of the CDR2 region of AHP15662-VHH4: SEQ ID NO: 18
The amino acid sequence of the CDR3 region of AHP15662-VHH4: SEQ ID NO: 19
The DNA sequence of the heavy chain variable region of the single domain antibody AHP15662-VHH4: SEQ ID NO: 44
The amino acid sequence of the heavy chain variable region of the single domain antibody AHP15773-VHH4: SEQ ID NO: 45
The amino acid sequence of the CDR1 region of AHP15773-VHH4: SEQ ID NO: 27
The amino acid sequence of the CDR2 region of AHP15773-VHH4: SEQ ID NO: 28
The amino acid sequence of the CDR3 region of AHP15773-VHH4: SEQ ID NO: 29
The DNA sequence of the heavy chain variable region of the single domain antibody AHP15773-VHH4: SEQ ID NO: 46

### Example 6: SPR binding assay of humanized antibodies to human ROR1-related proteins

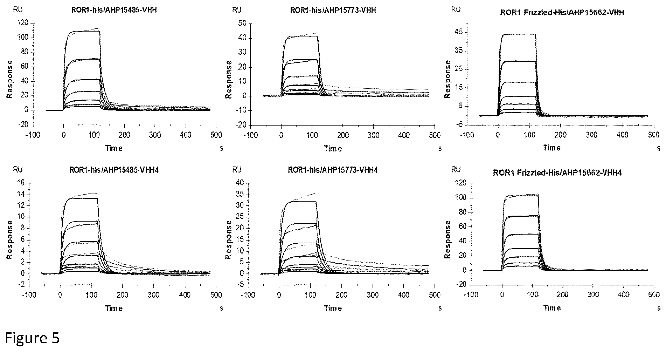

The affinity of purified antibodies for human ROR1-related proteins was determined separately using the surface plasmon resonance (SPR) biosensor Biacore T200 (GE Healthcare). The antibodies were immobilized on the sensor chip by the Fc capture method. Human ROR1-related proteins were used as analytes. Data for dissociation (kd) and association (ka) rate constants were obtained using the Biacore T200 evaluation software. The equilibrium dissociation constant (KD) was calculated from the ratio of kd to ka. The antibodies were sorted by their equilibrium dissociation constants, and humanized antibodies with no decrease or no more than a 3-fold decrease in affinity were selected for subsequent detection. The results of the SPR affinity assay are as shown in Table 3 below, and the relevant sensorgrams are as shown in Figure 5. As determined by SPR affinity assay, the three groups of parental and humanized antibodies all exhibited comparable affinity levels for the human ROR1 antigen proteins, and their equilibrium dissociation constants (KDs) were all in the range of 10⁻⁷.

**Table 3. Affinity determination of humanized single domain antibodies**

| Ligand | Analyte | ka (1/Ms) | kd (1/s) | KD (M) | Rmax (RU) | Chi² (RU²) |
|---|---|---|---|---|---|---|
| AHP15485-VHH | ROR1-his | 2.25E+05 | 6.30E-02 | 2.80E-07 | 120.5 | 8 |
| AHP15485-VHH4 | ROR1-his | 2.14E+05 | 6.86E-02 | 3.20E-07 | 24.1 | 0.356 |
| AHP15773-VHH | ROR1-his | 1.55E+05 | 1.17E-01 | 7.57E-07 | 119.9 | 4.3 |
| AHP15773-VHH4 | ROR1-his | 1.84E+05 | 5.87E-02 | 3.19E-07 | 57.6 | 5.12 |
| AHP15662-VHH | ROR1 Frizzled-His | 3.45E+05 | 1.90E-01 | 5.50E-07 | 104.6 | 0.167 |
| AHP15662-VHH4 | ROR1 Frizzled-His | 4.49E+05 | 1.11E-01 | 2.47E-07 | 151.5 | 1.18 |

### Example 7: Binding of humanized antibodies to cell lines expressing human and mouse ROR1

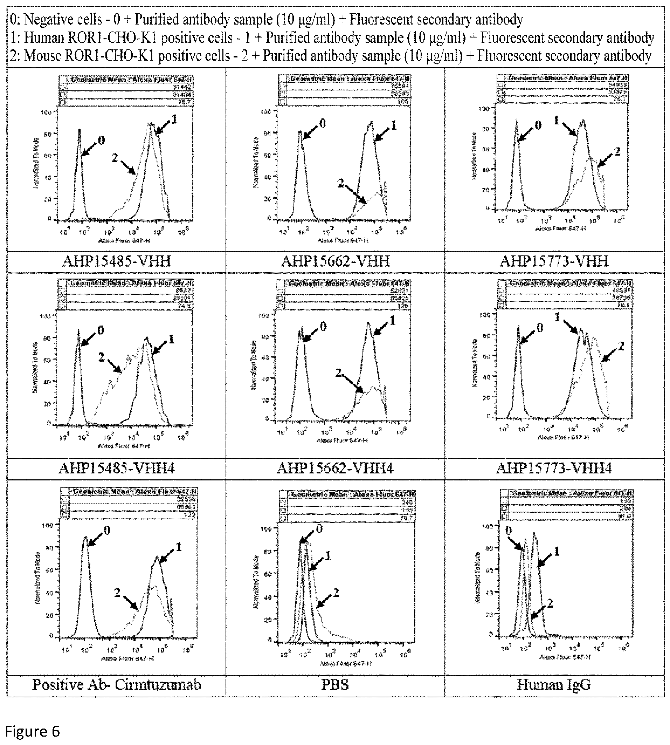

CHO cells expressing human and mouse ROR1 and parental cells as the negative control for detection were collected and washed 3 times with PBS. 2.5 X 10⁵ test cells and 100 ul of purified antibody at 10 µg/ml were added to a 96-well plate, and the plate was incubated at 4°C for 1 hour. The cells were then washed 3 times with PBS, 100 µl of iFluor-labeled goat anti-human IgG, an Fcy-specific fragment antibody, was added, and the plate was incubated at 4°C for 45 minutes. Finally, the cells were washed 3 times with PBS, and the signals were read using FACS BD Calibur. As shown in Figure 6, all the humanized antibodies bound to the ROR1-expressing CHO-K1/Human ROR1 stable cells and CHO-K1/Mouse ROR1 stable cells but did not bind to the CHO parental cells, and their binding was comparable to that of the corresponding parental samples.

### Example 8: Construction and detection of human ROR1-engineered cell lines

After the ROR1 DNA ORF sequence (the sequence was derived from NP_005003.2, and the amino acid sequence is as set forth in SEQ ID NO: 47) was synthesized, the DNA fragment of ROR1 ORF was ligated to the vector backbone pLVX-Puromycin using the Clone EZ (GenScript) technology. The ligation product was transformed into competent E. coli cells to obtain the plasmid pLVX-ROR1-puromycin.

Lentivirus packaging: Using a three-plasmid system, pLVX-ROR1-puromycin and two helper vector plasmids (psPAX2 and PMD2.G) were co-transfected into 293T cells. The supernatant of 293T cells was collected and ultracentrifuged so as to obtain the concentrated and purified viruses. The collected viruses were stored at -80°C.

Infecting cells: CHO-K1 cells (ECACC) were plated into a 6-well plate, 3 mL of medium was added, and the plate was incubated overnight. Before infection, the viruses were removed from the freezer and rapidly thawed in a water bath at 37°C. The original medium of the cells was removed, and 1/2 volume of fresh medium was added. The virus stock solution was then added to the cells, and the mixture was mixed evenly. After centrifugation and infection for 0.5 to 1 hour, the culture plate was incubated in an incubator at 37°C with 5% CO₂ for 24 hours. The day after infection (about 24 hours), the virus-containing medium solution was removed and exchanged to fresh complete medium solution, and the incubation was continued at 37°C.

Screening for puromycin resistance: The cell culture medium was added with 8 µg/ml of puromycin (Thermo Fisher, Cat# A11138-03), and exchanged to the complete medium solution containing puromycin every 2-3 days until the cells in the uninfected screening control group died completely under the screening pressure of puromycin. Continuous screening was carried out until stable cell lines were obtained.

FACS detection of ROR1 expression: A portion of the obtained stable cells was transferred to a FACS tube and centrifuged to remove the supernatant. PE anti-human ROR1 Antibody (BioLegend, Cat# 357804) was added, and the mixture was incubated at 4°C for 30 minutes. After 30 minutes, the supernatant was removed, the cells were resuspended in FACS buffer, and loaded onto the flow cytometer (BD FACS Celesta^{™}) to detect the expression level of ROR1.

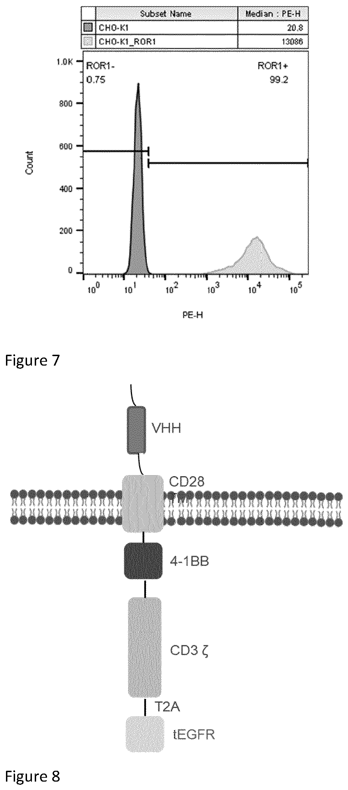

Selecting monoclones: The cell pool was subjected to limiting dilution into a 96-well plate. After 7 days, the 96-well plate was observed under a microscope, and wells containing monoclones were marked. The monoclonal cells were transferred to a 24-well plate and then expanded to a 6-well plate. The expression amount of CHO-K1/ROR1 monoclones was verified by the above steps after the expansion of the monoclones. The results of the best clone are as shown in Figure 7. ROR1 overexpression from CHO-K1/ROR1 was detected by FACS.

### Example 9: CAR vector structure

A second-generation CAR vector targeting human ROR1 was constructed in the present disclosure. As shown in Figure 8, the structure sequentially comprises, for example, the ROR1 VHH antibody sequence, the CD28 transmembrane region, the 4-1BB intracellular region sequence, the CD3zeta sequence, and the tEGFR sequence serving as the transduction indicator protein. The amino acid sequences of the CARs of the present disclosure (AHP15485 CAR, AHP15580 CAR, AHP15662 CAR, AHP15773 CAR, AHP15768 CAR, AHP16026 CAR, AHP15547 CAR, and AHP15776 CAR) and R12 CAR are as set forth in SEQ ID NOs: 56-64.

### Example 10: Construction of human ROR1-targeted CAR-Jurkat cells

In order to preliminarily verify the functional effects of ROR1 CARs, ROR1-CAR-Jurkat cells were constructed.

Jurkat/NFAT-Luc cells were constructed with the following construction steps: Jurkat cells (ATCC) were plated at 3 × 10⁵ cells per well into a 6-well plate. The NFAT-Luc virus concentrate was added for infection. On the third day (approximately 48 hours) after infection, Hygromycin B (Thermo Fisher, Cat# 10687-010) was used for resistance screening, and the Jurkat/NFAT-Luc cell pool was obtained.

Production of ROR1 CAR-concentrated viruses: The preparation of lentivirus referred to the lentivirus packaging technology in the construction of the ROR1-engineered cell line in Example 8 herein.

Preparation of ROR1-CAR-Jurkat cells: The Jurkat/NFAT-Luc cell pool was infected with the ROR1 CAR-concentrated viruses at MOI = 10. At 72 h after infection, the expression amount of the CARs was detected by FACS. As shown in Table 4, the expression of the CAR transduction indicator protein EGFR (PE-EGFR Antibody, Novus, NBP2-75903PE) was detected by FACS, and the results showed that the transduction efficiencies were all greater than 99%.

**Table 4. Detection of positive rate of human ROR1-targeted CAR-Jurkat cells**

| CAR-T No. | CAR expression % |
|---|---|
| UnT | 2.85% |
| AHP15485 CAR | 100% |
| AHP15580 CAR | 100% |
| AHP15662 CAR | 99.9% |
| AHP15773 CAR | 100% |
| AHP15768 CAR | 99.8% |
| AHP16026 CAR | 100% |
| AHP15547 CAR | 99.9% |
| AHP15776 CAR | 99.8% |
| R12 CAR | 99.9% |

### Example 11: Specific activation of human ROR1-targeted CAR-Jurkat cells by target cells

In order to verify whether the ROR1-CARs constructed in the present disclosure can be specifically activated by ROR1-positive cells, ROR1-CAR Jurkat cells with 8 different ROR1 VHH sequences (SEQ ID NOs: 56-63) were constructed, and R12 CAR-Jurkat cells constructed with R12 ScFv (SEQ ID NO: 64) (Patent Publication No.: WO 2014/031687 A1) were used as the positive reference for the experiment. After these cells were co-incubated with CHO-K1/ROR1 cells, respectively, the fluorescence signal value and the cytokine IL-2 secretion were detected.

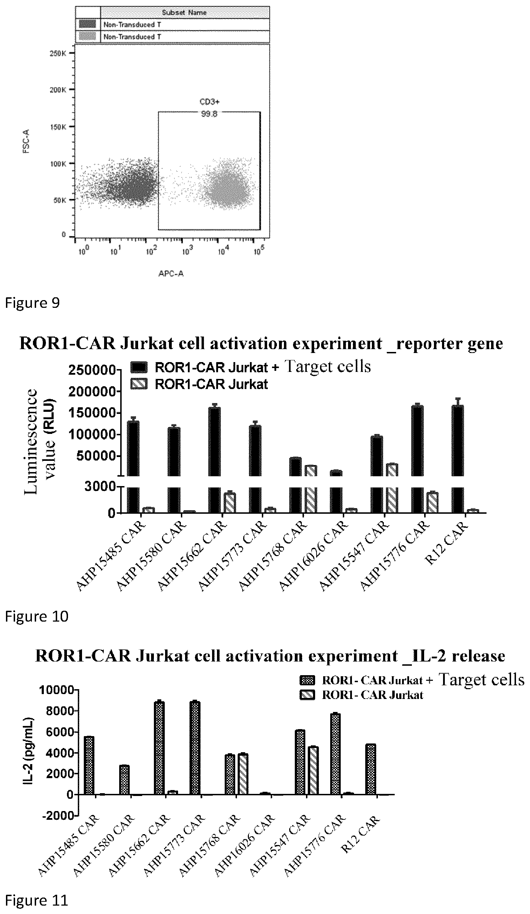

Evaluation of the activation effect of ROR1-CARs on Jurkat cells based on the reporter gene method: The target cells CHO-K1/ROR1 were collected and resuspended in the complete medium F12K (Gibco, Cat# 21127-022) + 10% FBS (Gibco, Cat# 10091-148), and the cell suspension was adjusted to a density of 2E5 cells/mL. Subsequently, 50 µL of target cell suspension was plated into a 96-well plate. The culture plate containing the target cells was transferred to an incubator at 37°C with 5% CO₂ and incubated overnight. On the next day, the effector cells ROR1-CAR Jurkat were collected and resuspended in the complete medium 1640 (Gibco, Cat# 22400-089) + 10% FBS (Gibco, Cat# 10091-148), and the cell suspension was then adjusted to a density of 4E5 cells/mL. The target cell culture plate that had been incubated overnight was removed from the incubator, the previous culture medium was discarded, and 100 µL of effector cell suspension was transferred. Subsequently, the culture plate was transferred to the incubator at 37°C with 5% CO₂ and incubated for another 6 h. The culture plate was removed, and the detection reagent Fire-Lumi^{™} Luciferase Assay Kit (GenScript, Cat# L00877C) was added. After the plate was incubated at room temperature for 5 minutes, the Luminescence signal value was detected by a microplate reader (PHERAStar FSX, BMG). The results are as shown in Figure 10 and Table 5. Among the 8 sequence molecules, the cells with molecular sequences of AHP15485, AHP15580, AHP15662, AHP15773, and AHP15776 showed relatively stronger activation effects on Jurkat cells, and the signal ratios were closer to that of the positive control R12 ScFv.

**Table 5. Signal ratios of activation of ROR1-CAR Jurkat cells _ reporter gene method**

| ROR1 CAR | S/B signal ratio |
|---|---|
| AHP15485 CAR | 233.74 |
| AHP15580 CAR | 530.90 |
| AHP15662 CAR | 73.87 |
| AHP15773 CAR | 252.80 |
| AHP15768 CAR | 1.66 |
| AHP16026 CAR | 32.19 |
| AHP15547 CAR | 3.18 |
| AHP15776 CAR | 73.57 |
| R12 CAR | 458.47 |

Evaluation of the activation effect of ROR1-CARs on Jurkat cells based on the cytokine IL-2 secretion level: The target cells CHO-K1/ROR1 were collected and resuspended in the complete medium F12K (Gibco, Cat# 21127-022) + 10% FBS (Gibco, Cat# 10091-148), and the cell suspension was adjusted to a density of 2E5 cells/mL. Subsequently, 50 µL of target cell suspension was plated into a 96-well plate. The culture plate containing the target cells was transferred to an incubator at 37°C with 5% CO₂ and incubated overnight. On the next day, the effector cells ROR1-CAR Jurkat were collected and resuspended in the complete medium 1640 (Gibco, Cat# 22400-089) + 10% FBS (Gibco, Cat# 10091-148), and the cell suspension was then adjusted to a density of 4E5 cells/mL. The target cell culture plate that had been cultured overnight was removed from the incubator, the previous culture medium was discarded, and 100 µL of effector cell suspension was transferred. Subsequently, the culture plate was transferred to the incubator at 37°C with 5% CO₂ and incubated for another 24 h. The culture supernatant was collected to detect the cytokine IL-2 secretion using the Human IL2 kit (Cisbio, Cat# 62HIL02PEH) according to its instructions, and the signal value was detected by a microplate reader (PHERAStar FSX, BMG). The results are as shown in Figure 11 and Table 6. The molecular sequences of AHP15485, AHP15580, AHP15662, AHP15773, and AHP15776 showed relatively stronger activation effects on T cells, and the signal ratios were closer to that of the positive control R12 ScFv.

**Table 6. Ratios of cytokine IL-2 secretion levels in ROR1-CAR-Jurkat cells**

| ROR1 CAR | S/B signal ratio |
|---|---|
| AHP15485 CAR | 12.98 |
| AHP15580 CAR | 7.37 |
| AHP15662 CAR | 12.11 |
| AHP15773 CAR | 20.95 |
| AHP15768 CAR | 0.98 |
| AHP16026 CAR | 1.37 |
| AHP15547 CAR | 1.31 |
| AHP15776 CAR | 13.57 |
| R12 CAR | 11.80 |

The ROR1-CAR-Jurkat cells exhibited fluorescent signal values and increased IL-2 secretion to varying degrees, indicating that the 8 ROR1-CAR-Jurkat cells constructed in the present disclosure could be specifically activated by ROR1-positive cells, and suggesting that the molecular sequences of AHP15485, AHP15580, AHP15662, AHP15773, and AHP15776 showed relatively stronger activation effects on T cells. The detection results of fluorescent signal values by the reporter gene method were consistent with the detection results of cytokine IL-2 secretion.

### Example 12: Construction and identification of ROR1-CAR-T cells

In order to further validate the function of each ROR1-CAR in primary T cells, ROR1-CAR-T cells with 8 different ROR1 VHH sequences were constructed by means of lentiviral transfection, and R12 CAR-T cells constructed with R12 ScFv were used as the positive reference for the experiment. The specific construction steps were as follows:
Activation of T cells: After the cryopreserved PBMCs (Sailybio) were thawed, the cells were resuspended in the complete medium AIM V (ThermoFisher, Cat# 31035025) containing 300 U/ml of rhlL-2 and counted. Subsequently, the T cells were activated with CD3/CD28 antibody magnetic beads (GenScript) at a ratio of 1 : 1 between the cells and the beads (the activation time point was recorded as D0, and the time points 1 day and 2 days after activation were recorded as D1 and D2, respectively). After the cells were co-incubated with the magnetic beads for 24 h, the activated T cells were obtained.

Lentivirus infection of T cells: The activated T cells were plated in a 24-well plate, and the cell density of each well was adjusted to 1E6 cells. CAR lentiviruses (MOI = 10) were added, with a total system of 400 µl. 16 µl of viral infection-promoting reagent HiTransG P (Genechem, Cat# REVG005) was also added. After 16 h of transfection, each well was refilled with 1 ml of medium for culture, and after 72 h of culture, CAR-T cells targeting ROR1 were obtained.

Detection of T cell proportion by flow cytometry: 3 days after transfection (D4 after activation), the proportion of CD3 was detected by FACS. The flow cytometry detection results are as shown in Figure 9. The proportion of CD3 was greater than 99%, suggesting that the proportion of T cells in the cell population was greater than 99%.

Detection of CAR cell proportion by flow cytometry: 3 days after transfection (D4 after activation), the transduction efficiency of the CARs was detected by FACS. Positive rate results for the CARs are as shown in Table 7. The transfection efficiencies of the CARs were all greater than 70%.

**Table 7. Detection of positive rate of ROR1-CAR-T**

| CAR-T No. | CAR expression % |
|---|---|
| UnT | 2.54% |
| AHP15485 CAR | 87.8% |
| AHP15580 CAR | 80.7% |
| AHP15662 CAR | 84.3% |
| AHP15773 CAR | 78.9% |
| AHP15768 CAR | 84.0% |
| AHP16026 CAR | 83.9% |
| AHP15547 CAR | 82.7% |
| AHP15776 CAR | 73.1% |
| R12 CAR | 84.5% |

### Example 13: Experiment of in vitro killing of ROR1-CAR-T cells against ROR1-positive target cells

The target cells CHO-K1/ROR1/Luc (Nanjing Probio, Cat# RD00949) were collected and resuspended in the complete medium 1640 (Gibco, Cat# 22400-089) + 10% FBS (Gibco, Cat# 10091-148), and the cell suspension was adjusted to a density of 1E5 cells/mL. Subsequently, 50 µL of target cell suspension was plated into a 96-well plate. The ROR1-CAR-T cells were collected and resuspended in the complete medium 1640 (Gibco, Cat# 22400-089). The suspension was adjusted to a theoretical density of 2E6 cells/mL according to the CAR positive rates of different transduction molecules (see Table 7). Subsequently, 50 µL of ROR1-CAR-T cell suspension and 50 µL of 1% Triton X-100 (J&K Scientific Ltd., Cat# 993361) (as the maximum killing signal value) were transferred to the 96-well plate plated with the target cells. Subsequently, the culture plate was transferred to an incubator at 37°C with 5% CO₂ and incubated for 24 h. The culture plate that had been incubated for 24 h was removed, and the detection reagent Fire-Lumi^{™} Luciferase Assay Kit (GenScript, Cat# L00877C) was added. After the plate was incubated at room temperature for 5 minutes, the Luminescence signal value was detected by a microplate reader (PHERAStar FSX, BMG).

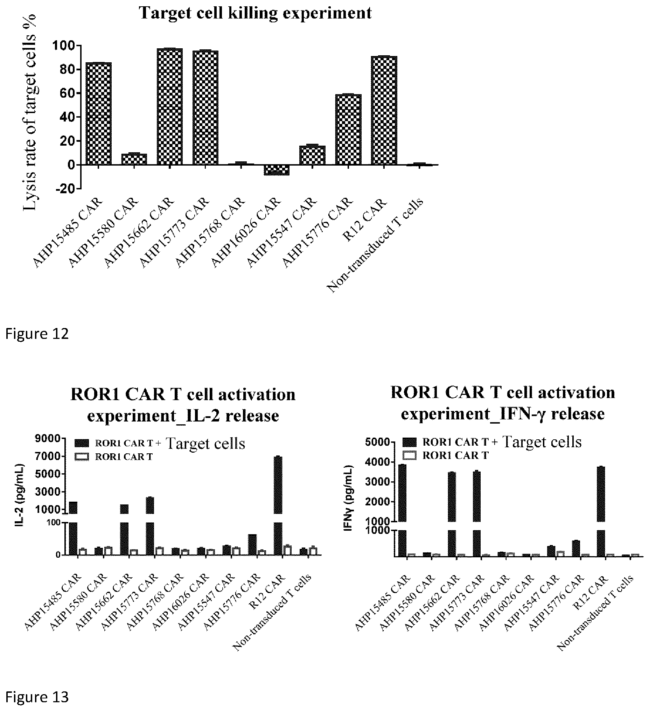

The killing values of ROR1-CAR-T with different transduction molecules were calculated using the killing calculation formula: % Cytotoxicity = 100 * (1 - (RLU _{Experimental} - RLU _{Min}) / (RLU _{UnT} - RLU _{Min})). The results are as shown in Figure 12 and Table 8. The CAR T cells with molecular sequences of AHP15485, AHP15662, and AHP15773 exhibited killing effects to the same degree as the positive control R12 CAR T (AHP15485 % Cytotoxicity = 84.86%, AHP15662 % Cytotoxicity = 96.69%, AHP15773 % Cytotoxicity = 94.82%, and R12 CAR % Cytotoxicity = 90.28%).

RLU _{Experimental}: the signal value of ROR1-CAR T cells + target cells CHO-K1/ROR1/Luc; RLU _{UnT}: the signal value of non-transduced T cells + target cells CHO-K1/ROR1/Luc, where the signal value was the highest since the target cells were not specifically killed by CAR-T cells; RLU _{Min}: the signal value of 1% Triton X-100 + target cells CHO-K1/ROR1/Luc, where the signal value was the lowest since the target cells were completely lysed by Triton X-100 (RLU _{Min} in the killing calculation formula).

**Table 8. Killing values of ROR1-CAR-T cells against target cells CHO-K1/ROR1/Luc**

| ROR1 CAR | % Cytotoxicity |
|---|---|
| AHP15485 CAR | 84.86 |
| AHP15580 CAR | 8.36 |
| AHP15662 CAR | 96.69 |
| AHP15773 CAR | 94.82 |
| AHP15768 CAR | 0.40 |
| AHP16026 CAR | -8.02 |
| AHP15547 CAR | 15.27 |
| AHP15776 CAR | 58.30 |
| R12 CAR | 90.28 |
| Non-transduced T cells | 0.00 |

### Example 14: Experiment of cytokine release of ROR1-CAR-T cells against ROR1-positive target cells

The target cells CHO-K1/ROR1/Luc were collected and resuspended in the complete medium 1640 (Gibco, Cat# 22400-089) + 10% FBS (Gibco, Cat# 10091-148), and the cell suspension was adjusted to a density of 5E4 cells/mL. Subsequently, 100 µL of target cell suspension was plated into a 96-well plate. The ROR1-CAR T cells with different transduction molecules were collected and resuspended in the complete medium 1640 (Gibco, Cat# 22400-089) + 10% FBS (Gibco, Cat# 10091-148). The suspension was adjusted to a theoretical density of 1E6 cells/mL according to the CAR positive rates of different transduction molecules (see Table 7). Subsequently, 100 µL of ROR1-CAR-T suspension was transferred to the 96-well plate plated with the target cells. Subsequently, the culture plate was transferred to an incubator at 37°C with 5% CO₂ and incubated for 24 h. The culture plate that had been incubated for 24 h was removed. The culture supernatant was collected to detect the cytokine IL-2 and IFN-y secretion using the Human IL2 kit (Cisbio, Cat# 62HIL02PEH) and Human IFN gamma kit (Cisbio, Cat# 62HIFNGPEH) according to their instructions, respectively, and the signal value was detected by a microplate reader (PHERAStar FSX, BMG). The results are as shown in Figure 13 and Tables 9 and 10. Among the 8 molecular sequences, the CAR T cells with molecular sequences of AHP15485, AHP15662, and AHP15773 showed relatively higher cytokine secretion levels, which were comparable to the secretion levels of the positive control R12 CAR T (comparison of IL-2 secretion levels: AHP15485 S/B ratio = 109.00, AHP15662 S/B ratio = 99.87, AHP15773 S/B ratio = 107.49, and R12 CAR S/B ratio = 265.67; and comparison of IFN-y secretion levels: AHP15485 S/B ratio = 46.87, AHP15662 S/B ratio = 48.45, AHP15773 S/B ratio = 58.03, and R12 CAR S/B ratio = 51.33). The detection results were consistent with the killing effects.

**Table 9. Cytokine IL-2 release of ROR1-CAR-T cells against target cells CHO-K1/ROR1/Luc**

| ROR1 CAR | S/B ratio |
|---|---|
| AHP15485 CAR | 109.00 |
| AHP15580 CAR | 0.89 |
| AHP15662 CAR | 99.78 |
| AHP15773 CAR | 107.49 |
| AHP15768 CAR | 1.40 |
| AHP16026 CAR | 1.34 |
| AHP15547 CAR | 1.26 |
| AHP15776 CAR | 5.08 |
| R12 CAR | 265.67 |
| Non-transduced T cells | 0.83 |

**Table 10. Cytokine IFN-y release of ROR1-CAR-T cells against target cells CHO-K1/ROR1/Luc**

| ROR1 CAR | S/B ratio |
|---|---|
| AHP15485 CAR | 46.87 |
| AHP15580 CAR | 1.63 |
| AHP15662 CAR | 48.45 |
| AHP15773 CAR | 58.03 |
| AHP15768 CAR | 1.24 |
| AHP16026 CAR | 0.92 |
| AHP15547 CAR | 2.15 |
| AHP15776 CAR | 8.23 |
| R12 CAR | 51.33 |
| Non-transduced T cells | 0.62 |

### Example 15: Affinity maturation and recombinant validation of humanized single domain antibodies

The humanized single domain antibodies were synthesized in the prokaryotic soluble expression vector of Probio, and prokaryotic expression and binding verification were carried out. After confirming that the expression was normal, the prokaryotic soluble expression plasmid of the single domain antibody would be used as a template for the subsequent mutation library. A precise saturation mutation library of the CDR regions of the single domain antibody was synthesized in Genscript. After the bacterial solution sample of the synthesized library was received, an ELISA binding assay with the antigen protein was carried out. According to the results of the ELISA assay, the clones with improved binding values were selected for SPR ranking assay. Based on the ratio of the dissociation constants between the sample and the humanized single domain antibody sample in the SPR binding assay results, the clones with significant improvement were selected for Sanger sequencing to determine the specific mutation sites of the improved clones and the amino acids after mutation.

According to the mutation status of the improved clones in the precise saturation mutation library, the combinatorial mutations and the relevant primers for library construction for the second round were designed. The combinatorial mutation library construction was performed still using the prokaryotic soluble expression plasmid of the humanized single domain antibody as the template. The subsequent screening process was the same as that for the precise saturation mutation library. Finally, the top 5 or 7 clones ranked by the improvement fold in affinity were selected for recombinant verification. 5 clones with improved affinity (AHF22016, AHF22018, AHF22013, AHP15485-VHH4-Variant1, and AHP15485-VHH4-Variant2) were obtained from the humanized antibody AHP15485 through affinity maturation screening, and were subjected to eukaryotic expression and purification, as well as subsequent affinity detection at various concentrations. 7 clones (AHP15662-VHH4-Variant1, AHP15662-VHH4-Variant2, AHP15662-VHH4-Variant3, AHF21711, AHF21719, AHF21720, and AHF21721) were obtained from AHP15662-VHH4. After the clones with improved affinity were subjected to eukaryotic recombinant expression and purification, the affinity of purified antibodies for human ROR1-related proteins was determined separately using the surface plasmon resonance (SPR) biosensor Biacore T200 (GE Healthcare). Sequences of the clones with improved affinity are listed in detail below, and the specific affinity detection method was the same as Example 6.
The amino acid sequence of the heavy chain variable region of the single domain antibody AHF22013: SEQ ID NO: 65
The amino acid sequence of the CDR1 region of AHF22013: SEQ ID NO: 66
The amino acid sequence of the CDR2 region of AHF22013: SEQ ID NO: 3
The amino acid sequence of the CDR3 region of AHF22013: SEQ ID NO: 67
The amino acid sequence of the heavy chain variable region of the single domain antibody AHF22016: SEQ ID NO: 68
The amino acid sequence of the CDR1 region of AHF22016: SEQ ID NO: 66
The amino acid sequence of the CDR2 region of AHF22016: SEQ ID NO: 3
The amino acid sequence of the CDR3 region of AHF22016: SEQ ID NO: 69
The amino acid sequence of the heavy chain variable region of the single domain antibody AHF22018: SEQ ID NO: 70
The amino acid sequence of the CDR1 region of AHF22018: SEQ ID NO: 66
The amino acid sequence of the CDR2 region of AHF22018: SEQ ID NO: 3
The amino acid sequence of the CDR3 region of AHF22018: SEQ ID NO: 71
The amino acid sequence of the heavy chain variable region of the single domain antibody AHP15485-VHH4-Variant1: SEQ ID NO: 72
The amino acid sequence of the CDR1 region of AHP15485-VHH4-Variant1: SEQ ID NO: 2
The amino acid sequence of the CDR2 region of AHP15485-VHH4-Variant1: SEQ ID NO: 3
The amino acid sequence of the CDR3 region of AHP15485-VHH4-Variant1: SEQ ID NO: 4
The amino acid sequence of the heavy chain variable region of the single domain antibody AHP15485-VHH4-Variant2: SEQ ID NO: 73
The amino acid sequence of the CDR1 region of AHP15485-VHH4-Variant2: SEQ ID NO: 66
The amino acid sequence of the CDR2 region of AHP15485-VHH4-Variant2: SEQ ID NO: 3
The amino acid sequence of the CDR3 region of AHP15485-VHH4-Variant2: SEQ ID NO: 4
The amino acid sequence of the heavy chain variable region of the single domain antibody AHP15662-VHH4-Variant1: SEQ ID NO: 74
The amino acid sequence of the CDR1 region of AHP15662-VHH4-Variant1: SEQ ID NO: 75
The amino acid sequence of the CDR2 region of AHP15662-VHH4-Variant1: SEQ ID NO: 76
The amino acid sequence of the CDR3 region of AHP15662-VHH4-Variant1: SEQ ID NO: 19
The amino acid sequence of the heavy chain variable region of the single domain antibody AHP15662-VHH4-Variant2: SEQ ID NO: 77
The amino acid sequence of the CDR1 region of AHP15662-VHH4-Variant2: SEQ ID NO: 78
The amino acid sequence of the CDR2 region of AHP15662-VHH4-Variant2: SEQ ID NO: 76
The amino acid sequence of the CDR3 region of AHP15662-VHH4-Variant2: SEQ ID NO: 19
The amino acid sequence of the heavy chain variable region of the single domain antibody AHP15662-VHH4-Variant3: SEQ ID NO: 79
The amino acid sequence of the CDR1 region of AHP15662-VHH4-Variant3: SEQ ID NO: 80
The amino acid sequence of the CDR2 region of AHP15662-VHH4-Variant3: SEQ ID NO: 76
The amino acid sequence of the CDR3 region of AHP15662-VHH4-Variant3: SEQ ID NO: 19
The amino acid sequence of the heavy chain variable region of the single domain antibody AHF21711: SEQ ID NO: 81
The amino acid sequence of the CDR1 region of AHF21711: SEQ ID NO: 82
The amino acid sequence of the CDR2 region of AHF21711: SEQ ID NO: 83
The amino acid sequence of the CDR3 region of AHF21711: SEQ ID NO: 19
The amino acid sequence of the heavy chain variable region of the single domain antibody AHF21719: SEQ ID NO: 84
The amino acid sequence of the CDR1 region of AHF21719: SEQ ID NO: 85
The amino acid sequence of the CDR2 region of AHF21719: SEQ ID NO: 76
The amino acid sequence of the CDR3 region of AHF21719: SEQ ID NO: 19
The amino acid sequence of the heavy chain variable region of the single domain antibody AHF21720: SEQ ID NO: 86
The amino acid sequence of the CDR1 region of AHF21720: SEQ ID NO: 85
The amino acid sequence of the CDR2 region of AHF21720: SEQ ID NO: 87
The amino acid sequence of the CDR3 region of AHF21720: SEQ ID NO: 19
The amino acid sequence of the heavy chain variable region of the single domain antibody AHF21721: SEQ ID NO: 88
The amino acid sequence of the CDR1 region of AHF21721: SEQ ID NO: 89
The amino acid sequence of the CDR2 region of AHF21721: SEQ ID NO: 90
The amino acid sequence of the CDR3 region of AHF21721: SEQ ID NO: 19

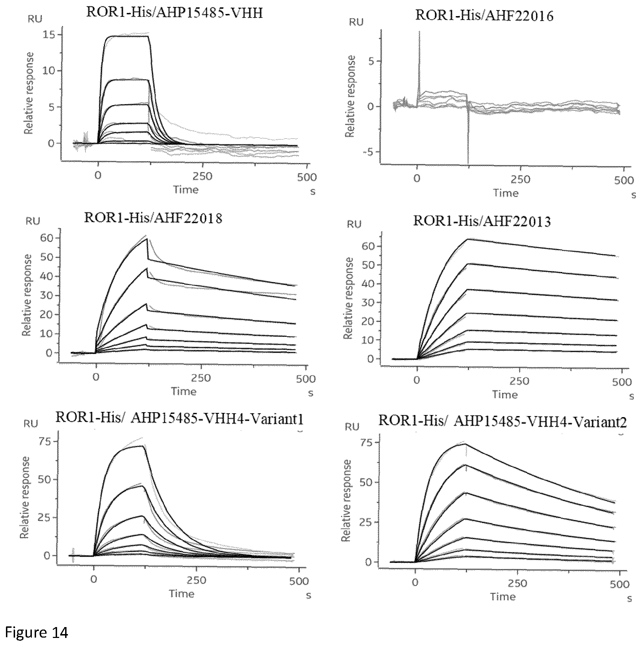

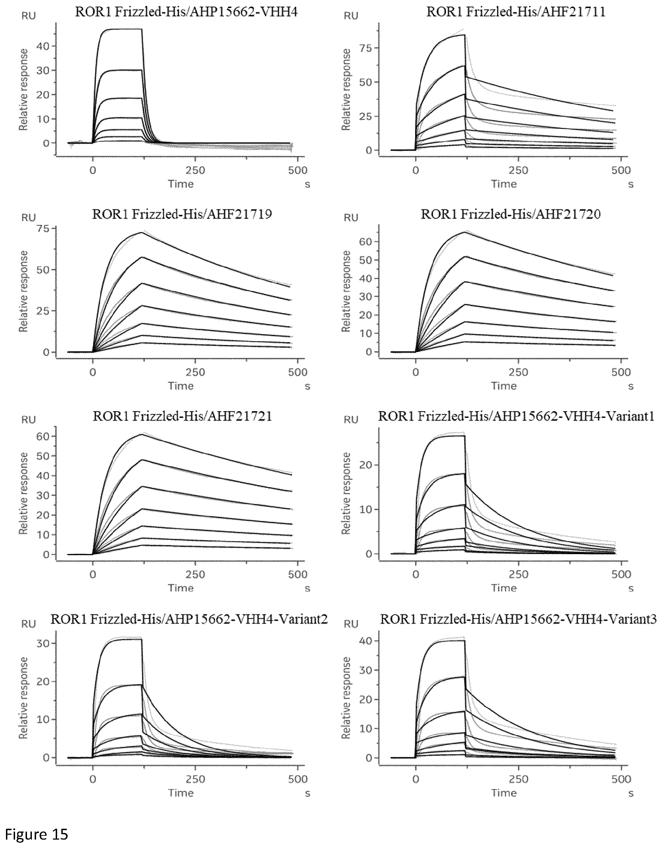

The results are as shown in Tables 11-12 and Figures 14-15. After affinity maturation, the affinity of the humanized single domain antibody AHP15485-VHH4 was improved to a maximum level of 10⁻⁹; and the affinity of the humanized single domain antibody AHP15662-VHH4 was improved to a maximum level of 10⁻⁸.

**Table 11. Affinity determination of AHP15485-VHH4 affinity-matured antibodies**

| Ligand | Analyte | Chi² (RU²) | ka (1/Ms) | kd (1/s) | KD (M) | Rmax (RU) |
|---|---|---|---|---|---|---|
| AHP15485-VHH4 | ROR1-his | 5.95E-01 | 1.89E+05 | 5.47E-02 | 2.90E-07 | 15.3 |
| AHF22016 | ROR1-his | NA | NA | NA | NA | NA |
| AHF22018 | ROR1-his | 7.60E-01 | 4.35E+04 | 8.84E-04 | 2.03E-08 | 57.6 |
| AHF22013 | ROR1-his | 1.69E-01 | 4.20E+04 | 3.74E-04 | 8.91E-09 | 64.7 |
| AHP15485-VHH4-Variant1 | ROR1-his | 3.26E+00 | 8.31E+04 | 1.53E-02 | 1.84E-07 | 78.3 |
| AHP15485-VHH4-Variant2 | ROR1-his | 3.73E-01 | 8.40E+04 | 1.80E-03 | 2.14E-08 | 80.2 |

**Table 12. Affinity determination of AHP15662-VHH4 affinity-matured antibodies**

| Ligand | Analyte | Chi² (RU²) | ka (1/Ms) | kd (1/s) | KD (M) | Rmax (RU) |
|---|---|---|---|---|---|---|
| AHP15662-VHH4 | ROR1 Frizzled-His | 3.26E-01 | 3.50E+05 | 1.03E-01 | 2.94E-07 | 76.4 |
| AHP15662-VHH4-Variant1 | ROR1 Frizzled-His | 7.68E-01 | 1.51E+05 | 7.01E-03 | 4.63E-08 | 27.1 |
| AHP15662-VHH4-Variant2 | ROR1 Frizzled-His | 1.12E+00 | 1.59E+05 | 1.19E-02 | 7.53E-08 | 39.3 |
| AHP15662-VHH4-Variant3 | ROR1 Frizzled-His | 1.80E+00 | 1.57E+05 | 6.20E-03 | 3.95E-08 | 61.6 |
| AHF21711 | ROR1 Frizzled-His | 4.96E+00 | 9.01E+04 | 1.74E-03 | 1.93E-08 | 62.6 |
| AHF21719 | ROR1 Frizzled-His | 8.39E-01 | 7.81E+04 | 1.69E-03 | 2.16E-08 | 75.5 |
| AHF21720 | ROR1 Frizzled-His | 5.71E-01 | 7.36E+04 | 1.24E-03 | 1.69E-08 | 66.2 |
| AHF21721 | ROR1 Frizzled-His | 4.90E-01 | 7.25E+04 | 1.13E-03 | 1.56E-08 | 62.8 |

The information of the amino acid and nucleotide sequences mentioned herein is as follows.
**The amino acid sequence of the heavy chain variable region of the single domain antibody AHP15485: SEQ ID NO: 1** 
EVQLVESGGGLAQAGGSLSLSCTASGRSFSNFQMGWFRQAPGKEREFVAASGWSGGPTYYADSVKGRFTIS
TDDSKKTMYLQMNSLKPEDTAAYYCYEDRVLSGRPVRYWGRGTQVTVSS
**The amino acid sequence of the CDR1 region of AHP15485: SEQ ID NO: 2**
   GRSFSNFQ
**The amino acid sequence of the CDR2 region of AHP15485: SEQ ID NO: 3**
   SGWSGG PT
**The amino acid sequence of the CDR3 region of AHP15485: SEQ ID NO: 4**
   YEDRVLSGRPVRY
**The DNA sequence of the heavy chain variable region of the single domain antibody AHP15485: SEQ ID NO: 5** 
GAAGTGCAGCTGGTGGAAAGCGGCGGCGGCCTGGCGCAGGCGGGCGGCAGCCTGAGCCTGAGCTGCA
CCGCGAGCGGCCGTAGCTTTAGCAACTTTCAGATGGGCTGGTTTCGTCAGGCGCCGGGCAAAGAACGTG
AATTTGTGGCGGCGAGCGGCTGGAGCGGCGGCCCGACCTATTATGCGGATAGCGTGAAAGGCCGTTTTA
CCATTAGCACCGATGATAGCAAAAAAACCATGTATCTGCAGATGAACAGCCTGAAACCGGAAGATACCG
CGGCGTATTATTGCTATGAAGATCGTGTGCTGAGCGGCCGTCCGGTGCGTTATTGGGGCCGTGGCACCC
AGGTGACCGTGAGCAGC
**The amino acid sequence of the heavy chain variable region of the single domain antibody AHP15547: SEQ ID NO: 6** 
AVQLVDSGGGSVQPGESLTLSCTGSARFLSNYGMGWYRQFPGKMREMVAYETNRGNKRYAESVKGRFRIA
RDAAKTTVYLEMNSLKPEDTAVYYCNAESSGWGRRNYWGQGTQVTVSS
**The amino acid sequence of the CDR1 region of AHP15547: SEQ ID NO: 7**
   ARFLSNYG
**The amino acid sequence of the CDR2 region of AHP15547: SEQ ID NO: 8**
   ETNRGNK
**The amino acid sequence of the CDR3 region of AHP15547: SEQ ID NO: 9**
   NAESSGWGRRNY
**The DNA sequence of the heavy chain variable region of the single domain antibody AHP15547: SEQ ID NO: 10** 
GCGGTGCAGCTGGTGGATAGCGGCGGCGGCAGCGTGCAGCCGGGCGAAAGCCTGACCCTGAGCTGCAC
CGGCAGCGCGTTTCTGAGCAACTATGGCATGGGCTGGTATCGTCAGTTTCCGGGCAAAATGCGTGA
AATGGTGGCGTATGAAACCAACCGTGGCAACAAACGTTATGCGGAAAGCGTGAAAGGCCGTTTTCGTAT
TGCGCGTGATGCGGCGAAAACCACCGTGTATCTGGAAATGAACAGCCTGAAACCGGAAGATACCGCGGT
GTATTATTGCAACGCGGAAAGCAGCGGCTGGGGCCGTCGTAACTATTGGGGCCAGGGCACCCAGGTGA
CCGTGAGCAGC
**The amino acid sequence of the heavy chain variable region of the single domain antibody AHP15580: SEQ ID NO: 11** 
QVQLVESGGGVVQPGGSLRLSCAASGFVLRVNTVSWYRQAPGKQRELVALITSESNEYYDDSVKGRFTISRD
NAKNTIYLHMNSLKPEDTGVYFCNFITTWGQGTQVTVSS
**The amino acid sequence of the CDR1 region of AHP15580: SEQ ID NO: 12**
   GFVLRVNT
**The amino acid sequence of the CDR2 region of AHP15580: SEQ ID NO: 13**
   ITSESNE
**The amino acid sequence of the CDR3 region of AHP15580: SEQ ID NO: 14**
   NFITT
**The DNA sequence of the heavy chain variable region of the single domain antibody AHP15580: SEQ ID NO: 15** 
CAGGTGCAGCTGGTGGAAAGCGGCGGCGGCGTGGTGCAGCCGGGCGGCAGCCTGCGTCTGAGCTGCG
CGGCGAGCGGCTTTGTGCTGCGTGTGAACACCGTGAGCTGGTATCGTCAGGCGCCGGGCAAACAGCGT
GAACTGGTGGCGCTGATTACCAGCGAAAGCAACGAATATTATGATGATAGCGTGAAAGGCCGTTTTACC
ATTAGCCGTGATAACGCGAAAAACACCATTTATCTGCATATGAACAGCCTGAAACCGGAAGATACCGGC
GTGTATTTTTGCAACTTTATTACCACCTGGGGCCAGGGCACCCAGGTGACCGTGAGCAGC
**The amino acid sequence of the heavy chain variable region of the single domain antibody AHP15662: SEQ ID NO: 16** 
EVQLVESGGGLVQPGGTLRLSCAASGSISRFYAVGWYRQAPGKQRELVARISSGGSTYYADSVKGRFTISINNS
KNTVYLQMNNLKPEDTAVYYCNARNLWGQGTQVTVSS
**The amino acid sequence of the CDR1 region of AHP15662: SEQ ID NO: 17**
   GSISRFYA
**The amino acid sequence of the CDR2 region of AHP15662: SEQ ID NO: 18**
   ISSGGST
**The amino acid sequence of the CDR3 region of AHP15662: SEQ ID NO: 19**
   NARNL
**The DNA sequence of the heavy chain variable region of the single domain antibody AHP15662: SEQ ID NO: 20** 
GAAGTGCAGCTGGTGGAAAGCGGCGGCGGCCTGGTGCAGCCGGGCGGCACCCTGCGTCTGAGCTGCGC
GGCGAGCGGCAGCATTAGCCGTTTTTATGCGGTGGGCTGGTATCGTCAGGCGCCGGGCAAACAGCGTG
AACTGGTGGCGCGTATTAGCAGCGGCGGCAGCACCTATTATGCGGATAGCGTGAAAGGCCGTTTTACCA
TTAGCATTAACAACAGCAAAAACACCGTGTATCTGCAGATGAACAACCTGAAACCGGAAGATACCGCGG
TGTATTATTGCAACGCGCGTAACCTGTGGGGCCAGGGCACCCAGGTGACCGTGAGCAGC
**The amino acid sequence of the heavy chain variable region of the single domain antibody AHP15768: SEQ ID NO: 21** 
QVQLVESGGGSVQAGGSLRLSCAVSGRSISLYNMGWFRQAPGKEREFVSAINWSGGRTYYADSVKGRFTISR
NNAKNTMYLQMNSLKPEDTATYYCAVDVEGWFRVSGDAAGYDYWGQGTQVTVSS
**The amino acid sequence of the CDR1 region of AHP15768: SEQ ID NO: 22**
   GRSISLYN
**The amino acid sequence of the CDR2 region of AHP15768: SEQ ID NO: 23**
   INWSGGRT
**The amino acid sequence of the CDR3 region of AHP15768: SEQ ID NO: 24**
   AVDVEGWFRVSGDAAGYDY
**The DNA sequence of the heavy chain variable region of the single domain antibody AHP15768: SEQ ID NO: 25** 
CAGGTGCAGCTGGTGGAAAGCGGCGGCGGCAGCGTGCAGGCGGGCGGCAGCCTGCGTCTGAGCTGCG
CGGTGAGCGGCCGTAGCATTAGCCTGTATAACATGGGCTGGTTTCGTCAGGCGCCGGGCAAAGAACGTG
AATTTGTGAGCGCGATTAACTGGAGCGGCGGCCGTACCTATTATGCGGATAGCGTGAAAGGCCGTTTTA
CCATTAGCCGTAACAACGCGAAAAACACCATGTATCTGCAGATGAACAGCCTGAAACCGGAAGATACCG
CGACCTATTATTGCGCGGTGGATGTGGAAGGCTGGTTTCGTGTGAGCGGCGATGCGGCGGGCTATGATT
ATTGGGGCCAGGGCACCCAGGTGACCGTGAGCAGC
**The amino acid sequence of the heavy chain variable region of the single domain antibody AHP15773: SEQ ID NO: 26** 
EVQLVESGGGLVQPGGSLRLSCLASGSDFSDSAMGWYRQAPGKQREFVAAIGAGGITTYADSVKGRFTISRD
SAKNIVYLQMSSLSPEDTAVYFCNFAPGWYPPGYQYWGQGTQVTVSS
**The amino acid sequence of the CDR1 region of AHP15773: SEQ ID NO: 27**
   GSDFSDSA
**The amino acid sequence of the CDR2 region of AHP15773: SEQ ID NO: 28**
   IGAGGIT
**The amino acid sequence of the CDR3 region of AHP15773: SEQ ID NO: 29**
   N FAPGWYPPGYQY
**The DNA sequence of the heavy chain variable region of the single domain antibody AHP15773: SEQ ID NO: 30** 
GAAGTGCAGCTGGTGGAAAGCGGCGGCGGCCTGGTGCAGCCGGGCGGCAGCCTGCGTCTGAGCTGCCT
GGCGAGCGGCAGCGATTTTAGCGATAGCGCGATGGGCTGGTATCGTCAGGCGCCGGGCAAACAGCGTG
AATTTGTGGCGGCGATTGGCGCGGGCGGCATTACCACCTATGCGGATAGCGTGAAAGGCCGTTTTACCA
TTAGCCGTGATAGCGCGAAAAACATTGTGTATCTGCAGATGAGCAGCCTGAGCCCGGAAGATACCGCGG
TGTATTTTTGCAACTTTGCGCCGGGCTGGTATCCGCCGGGCTATCAGTATTGGGGCCAGGGCACCCAGGT
GACCGTGAGCAGC
**The amino acid sequence of the heavy chain variable region of the single domain antibody AHP15776: SEQ ID NO: 31** 
QVQLVESGGGLVQPGGSLTLSCAASQSISSIYLLGWYRQAPGKERESVARITTSGTTTYIADSVKGRFTLSRDNA
KNTMYLQMNSLKPEDTAVYYCNVPFLVATARGNAYWGQGTQVTVSS
**The amino acid sequence of the CDR1 region of AHP15776: SEQ ID NO: 32**
   QSISSIYL
**The amino acid sequence of the CDR2 region of AHP15776: SEQ ID NO: 33**
   ITTSGTTT
**The amino acid sequence of the CDR3 region of AHP15776: SEQ ID NO: 34**
   NVPFLVATARGNAY
**The DNA sequence of the heavy chain variable region of the single domain antibody AHP15776: SEQ ID NO: 35** 
CAGGTGCAGCTGGTGGAAAGCGGCGGCGGCCTGGTGCAGCCGGGCGGCAGCCTGACCCTGAGCTGCGC
GGCGAGCCAGAGCATTAGCAGCATTTATCTGCTGGGCTGGTATCGTCAGGCGCCGGGCAAAGAACGTGA
AAGCGTGGCGCGTATTACCACCAGCGGCACCACCACCTATATTGCGGATAGCGTGAAAGGCCGTTTTACC
CTGAGCCGTGATAACGCGAAAAACACCATGTATCTGCAGATGAACAGCCTGAAACCGGAAGATACCGCG
GTGTATTATTGCAACGTGCCGTTTCTGGTGGCGACCGCGCGTGGCAACGCGTATTGGGGCCAGGGCACC
CAGGTGACCGTGAGCAGC
**The amino acid sequence of the heavy chain variable region of the single domain antibody AHP16026: SEQ ID NO: 36** 
QVQLVESGGGLVQPGGSLRLSCAVLGSIEQINDINYYRWYRQPPGKQRDLVASINNSGELIYADSVKGRFFISR
DKYTVYLEMRSLKAEDTAVYYCNAQDRGEYWGRGTQVTVSS
**The amino acid sequence of the CDR1 region of AHP16026: SEQ ID NO: 37**
   GSIEQIND
**The amino acid sequence of the CDR2 region of AHP16026: SEQ ID NO: 38**
   VASINNSG
**The amino acid sequence of the CDR3 region of AHP16026: SEQ ID NO: 39**
   NAQDRGEY
**The DNA sequence of the heavy chain variable region of the single domain antibody AHP16026: SEQ ID NO: 40** 
CAGGTGCAGCTGGTGGAAAGCGGCGGCGGCCTGGTGCAGCCGGGCGGCAGCCTGCGTCTGAGCTGCGC
GGTGCTGGGCAGCATTGAACAGATTAACGATATTAACTATTATCGTTGGTATCGTCAGCCGCCGGGCAAA
CAGCGTGATCTGGTGGCGAGCATTAACAACAGCGGCGAACTGATTTATGCGGATAGCGTGAAAGGCCGT
TTTTTTATTAGCCGTGATAAATATACCGTGTATCTGGAAATGCGTAGCCTGAAAGCGGAAGATACCGCGG
TGTATTATTGCAACGCGCAGGATCGTGGCGAATATTGGGGCCGTGGCACCCAGGTGACCGTGAGCAGC
**The amino acid sequence of the heavy chain variable region of the single domain antibody AHP15485-VHH4: SEQ ID NO: 41** 
EVQLVESGGGLVQPGGSLRLSCAASGRSFSNFQMGWFRQAPGKEREFVAASGWSGGPTYYADSVKGRFTIS
TDNSKNTLYLQMNSLRAEDTAVYYCYEDRVLSGRPVRYWGQGTLVTVSS
**The amino acid sequence of the CDR1 region of AHP15485-VHH4: SEQ ID NO: 2**
   GRSFSNFQ
**The amino acid sequence of the CDR2 region of AHP15485-VHH4: SEQ ID NO: 3**
   SGWSGG PT
**The amino acid sequence of the CDR3 region of AHP15485-VHH4: SEQ ID NO: 4**
   YEDRVLSGRPVRY
**The DNA sequence of the heavy chain variable region of the single domain antibody AHP15485-VHH4: SEQ ID NO: 42** 
GAAGTGCAGCTGGTCGAGAGCGGCGGCGGCCTGGTTCAACCTGGAGGATCTCTGAGACTGTCTTGTGCT
GCTTCTGGCAGAAGCTTCAGCAACTTCCAGATGGGCTGGTTTAGACAGGCCCCTGGCAAAGAGCGGGAA
TTCGTGGCCGCCAGCGGCTGGTCCGGCGGACCAACATACTATGCCGACAGCGTGAAGGGCAGATTCACA
ATCAGCACCGACAATAGCAAGAACACCCTGTACCTGCAGATGAACAGCCTGCGGGCCGAGGACACCGCC
GTGTACTACTGCTACGAGGATAGAGTGCTGAGCGGCCGCCCCGTGCGGTACTGGGGCCAGGGCACCCTC
GTGACCGTGTCCAGC
**The amino acid sequence of the heavy chain variable region of the single domain antibody AHP15662-VHH4: SEQ ID NO: 43** 
EVQLVESGGGLVQPGGSLRLSCAASGSISRFYAVGWYRQAPGKQRELVARISSGGSTYYADSVKGRFTISIDNS
KNTLYLQMNSLRAEDTAVYYCNARNLWGQGTLVTVSS
**The amino acid sequence of the CDR1 region of AHP15662-VHH4: SEQ ID NO: 17**
   GSISRFYA
**The amino acid sequence of the CDR2 region of AHP15662-VHH4: SEQ ID NO: 18**
   ISSGGST
**The amino acid sequence of the CDR3 region of AHP15662-VHH4: SEQ ID NO: 19**
   NARNL
**The DNA sequence of the heavy chain variable region of the single domain antibody AHP15662-VHH4: SEQ ID NO: 44** 
GAAGTGCAACTGGTCGAGAGCGGCGGAGGACTGGTGCAGCCCGGCGGCTCTCTGAGACTGAGCTGCGC
CGCTAGCGGCAGCATCTCTAGATTCTACGCCGTGGGCTGGTATAGACAGGCCCCTGGCAAACAGCGGGA
ACTGGTGGCCAGAATCAGCTCTGGCGGAAGCACCTACTACGCTGATAGCGTGAAGGGCAGATTCACCAT
CTCCATCGACAACAGCAAGAACACCCTCTACCTGCAGATGAACAGCCTGCGGGCCGAGGACACCGCCGT
GTACTACTGTAATGCCCGGAACCTGTGGGGCCAGGGCACACTGGTTACAGTGTCCAGC
**The amino acid sequence of the heavy chain variable region of the single domain antibody AHP15773-VHH4: SEQ ID NO: 45** 
EVQLVESGGGLVQPGGSLRLSCAASGSDFSDSAMGWYRQAPGKQREFVAAIGAGGITTYADSVKGRFTISRD
NAKNTLYLQMNSLRAEDTAVYFCNFAPGWYPPGYQYWGQGTLVTVSS
**The amino acid sequence of the CDR1 region of AHP15773-VHH4: SEQ ID NO: 27**
   GSDFSDSA
**The amino acid sequence of the CDR2 region of AHP15773-VHH4: SEQ ID NO: 28**
   IGAGGIT
**The amino acid sequence of the CDR3 region of AHP15773-VHH4: SEQ ID NO: 29**
   N FAPGWYPPGYQY
**The DNA sequence of the heavy chain variable region of the single domain antibody AHP15773-VHH4: SEQ ID NO: 46** 
GAAGTGCAGCTGGTGGAAAGCGGCGGAGGACTGGTCCAACCTGGCGGATCTCTGAGACTGAGCTGCGC
CGCTTCTGGCTCCGATTTCAGCGACAGCGCCATGGGCTGGTACAGACAGGCCCCTGGCAAACAGAGAGA
GTTCGTGGCTGCTATCGGCGCCGGCGGCATCACCACCTACGCCGATAGCGTGAAGGGCAGATTCACAAT
CAGCCGGGACAACGCCAAGAACACCCTGTACCTGCAGATGAACAGCCTGCGGGCCGAGGACACCGCCGT
GTACTTTTGTAATTTCGCCCCCGGCTGGTATCCTCCAGGATACCAGTACTGGGGCCAGGGCACACTCGTG
ACCGTGTCCAGC
**The amino acid sequence of ROR1: SEQ ID NO: 47** 
MHRPRRRGTRPPLLALLAALLLAARGAAAQETELSVSAELVPTSSWNISSELNKDSYLTLDEPMNNITTSLGQT
AELHCKVSGNPPPTIRWFKNDAPVVQEPRRLSFRSTIYGSRLRIRNLDTTDTGYFQCVATNGKEVVSSTGVLFV
KFGPPPTASPGYSDEYEEDGFCQPYRGIACARFIGNRTVYMESLHMQGEIENQITAAFTMIGTSSHLSDKCSQF
AIPSLCHYAFPYCDETSSVPKPRDLCRDECEILENVLCQTEYIFARSNPMILMRLKLPNCEDLPQPESPEAANCIR
IGIPMADPINKNHKCYNSTGVDYRGTVSVTKSGRQCQPWNSQYPHTHTFTALRFPELNGGHSYCRNPGNQK
EAPWCFTLDENFKSDLCDIPACDSKDSKEKNKMEILYILVPSVAIPLAIALLFFFICVCRNNQKSSSAPVQRQPKH
VRGQNVEMSMLNAYKPKSKAKELPLSAVRFMEELGECAFGKIYKGHLYLPGMDHAQLVAIKTLKDYNNPQQ
WTEFQQEASLMAELHHPNIVCLLGAVTQEQPVCMLFEYINQGDLHEFLIMRSPHSDVGCSSDEDGTVKSSLD
HGDFLHIAIQIAAGMEYLSSHFFVHKDLAARNILIGEQLHVKISDLGLSREIYSADYYRVQSKSLLPIRWMPPEAI
MYGKFSSDSDIWSFGVVLWEIFSFGLQPYYGFSNQEVIEMVRKRQLLPCSEDCPPRMYSLMTECWNEIPSRR
PRFKDIHVRLRSWEGLSSHTSSTTPSGGNATTQTTSLSASPVSNLSNPRYPNYMFPSQGITPQGQIAGFIGPPIP
QNQRFIPINGYPIPPGYAAFPAAHYQPTGPPRVIQHCPPPKSRSPSSASGSTSTGHVTSLPSSGSNQEANIPLLP
HMSIPNHPGGMGITVFGNKSQKPYKIDSKQASLLGDANIHGHTESMISAEL
**Signal peptide: SEQ ID NO: 48**
   MLLLVTSLLLCELPHPAFLLIP
Linker (used in R12 CAR as a control): **SEQ ID NO: 49**
   (G4S)₃
**Hinge region: SEQ ID NO: 50**
   ESKYGPPCPPCP
**Transmembrane region: SEQ ID NO: 51**
   FWVLVVVGGVLACYSLLVTVAFIIFWV
**4-1BB: SEQ ID NO: 52**
   KRGRKKLLYIFKQPFMRPVQTTQEEDGCSCRFPEEEEGGCEL
**CD3ζ: SEQ ID NO: 53** 
RVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR
**T2A: SEQ ID NO: 54**
   LEGGGEGRGSLLTCGDVEENPGPR
**tEGFR: SEQ ID NO: 55** 
MLLLVTSLLLCELPHPAFLLIPRKVCNGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVSRGRECVDK
CNLLEGEPREFVENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
**AHP15485 CAR: SEQ ID NO: 56** 
MLLLVTSLLLCELPHPAFLLIPEVQLVESGGGLAQAGGSLSLSCTASGRSFSNFQMGWFRQAPGKEREFVAAS
GWSGGPTYYADSVKGRFTISTDDSKKTMYLQMNSLKPEDTAAYYCYEDRVLSGRPVRYWGRGTQVTVSSES
KYGPPCPPCPFWVLVVVGGVLACYSLLVTVAFIIFWVKRGRKKLLYIFKQPFMRPVQTTQEEDGCSCRFPEEEE
GGCELRVKFSRSADAPAYQQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPRLEGGGEGRGSLLTCGDVEENPGPR
MLLLVTSLLLCELPHPAFLLIPRKVCNGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVSRGRECVDK
CNLLEGEPREFVENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
**AHP15580 CAR: SEQ ID NO: 57** 
MLLLVTSLLLCELPHPAFLLIPQVQLVESGGGVVQPGGSLRLSCAASGFVLRVNTVSWYRQAPGKQRELVALIT
SESNEYYDDSVKGRFTISRDNAKNTIYLHMNSLKPEDTGVYFCNFITTWGQGTQVTVSSESKYGPPCPPCPFW
VLVVVGGVLACYSLLVTVAFIIFWVKRGRKKLLYIFKQPFMRPVQTTQEEDGCSCRFPEEEEGGCELRVKFSRSA
DAPAYQQGQNQLYNELNLGRREEYDVLDKRRGRDPEMGGKPRRKNPQEGLYNELQKDKMAEAYSEIGMK
GERRRGKGHDGLYQGLSTATKDTYDALHMQALPPRLEGGGEGRGSLLTCGDVEENPGPRMLLLVTSLLLCEL
PHPAFLLIPRKVCNGIGIGEFKDSLSINATNIKHFKNCTSISGDLHILPVAFRGDSFTHTPPLDPQELDILKTVKEIT
GFLLIQAWPENRTDLHAFENLEIIRGRTKQHGQFSLAVVSLNITSLGLRSLKEISDGDVIISGNKNLCYANTINWK
KLFGTSGQKTKIISNRGENSCKATGQVCHALCSPEGCWGPEPRDCVSCRNVSRGRECVDKCNLLEGEPREFVE
 
NSECIQCHPECLPQAMNITCTGRGPDNCIQCAHYIDGPHCVKTCPAGVMGENNTLVWKYADAGHVCHLCH
PNCTYGCTGPGLEGCPTNGPKIPSIATGMVGALLLLLVVALGIGLFM
**AHP15662 CAR: SEQ ID NO: 58** 
MLLLVTSLLLCELPHPAFLLIPEVQLVESGGGLVQPGGTLRLSCAASGSISRFYAVGWYRQAPGKQRELVARISS
GGSTYYADSVKGRFTISINNSKNTVYLQMNNLKPEDTAVYYCNARNLWGQGTQVTVSSESKYGPPCPPCPF
WVLVVVGGVLACYSLLVTVAFIIFWVKRGRKKLLYIFKQPFMRPVQTTQEEDGCSCRFPEEEEGGCELRVKFSR
SADAPAYQQGQNQLYNELNLGRREEYDVLDKRRGRDPEMGGKPRRKNPQEGLYNELQKDKMAEAYSEIGM
KGERRRGKGHDGLYQGLSTATKDTYDALHMQALPPRLEGGGEGRGSLLTCGDVEENPGPRMLLLVTSLLLCE
LPHPAFLLIPRKVCNGIGIGEFKDSLSINATNIKHFKNCTSISGDLHILPVAFRGDSFTHTPPLDPQELDILKTVKEI
TGFLLIQAWPENRTDLHAFENLEIIRGRTKQHGQFSLAVVSLNITSLGLRSLKEISDGDVIISGNKNLCYANTINW
KKLFGTSGQKTKIISNRGENSCKATGQVCHALCSPEGCWGPEPRDCVSCRNVSRGRECVDKCNLLEGEPREFV
ENSECIQCHPECLPQAMNITCTGRGPDNCIQCAHYIDGPHCVKTCPAGVMGENNTLVWKYADAGHVCHLC
HPNCTYGCTGPGLEGCPTNGPKIPSIATGMVGALLLLLVVALGIGLFM
**AHP15773 CAR: SEQ ID NO: 59** 
MLLLVTSLLLCELPHPAFLLIPEVQLVESGGGLVQPGGSLRLSCLASGSDFSDSAMGWYRQAPGKQREFVAAI
GAGGITTYADSVKGRFTISRDSAKNIVYLQMSSLSPEDTAVYFCNFAPGWYPPGYQYWGQGTQVTVSSESKY
GPPCPPCPFWVLVVVGGVLACYSLLVTVAFIIFWVKRGRKKLLYIFKQPFMRPVQTTQEEDGCSCRFPEEEEG
GCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPRLEGGGEGRGSLLTCGDVEENPGPR
MLLLVTSLLLCELPHPAFLLIPRKVCNGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVSRGRECVDK
CNLLEGEPREFVENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
**AHP15768 CAR: SEQ ID NO: 60** 
MLLLVTSLLLCELPHPAFLLIPQVQLVESGGGSVQAGGSLRLSCAVSGRSISLYNMGWFRQAPGKEREFVSAIN
WSGGRTYYADSVKGRFTISRNNAKNTMYLQMNSLKPEDTATYYCAVDVEGWFRVSGDAAGYDYWGQGTQ
VTVSSESKYGPPCPPCPFWVLVVVGGVLACYSLLVTVAFIIFWVKRGRKKLLYIFKQPFMRPVQTTQEEDGCSC
RFPEEEEGGCELRVKFSRSADAPAYQQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPPRLEGGGEGRGSLLTCGDVE
 
ENPGPRMLLLVTSLLLCELPHPAFLLIPRKVCNGIGIGEFKDSLSINATNIKHFKNCTSISGDLHILPVAFRGDSFT
HTPPLDPQELDILKTVKEITGFLLIQAWPENRTDLHAFENLEIIRGRTKQHGQFSLAVVSLNITSLGLRSLKEISDG
DVIISGNKNLCYANTINWKKLFGTSGQKTKIISNRGENSCKATGQVCHALCSPEGCWGPEPRDCVSCRNVSRG
RECVDKCNLLEGEPREFVENSECIQCHPECLPQAMNITCTGRGPDNCIQCAHYIDGPHCVKTCPAGVMGENN
TLVWKYADAGHVCHLCHPNCTYGCTGPGLEGCPTNGPKIPSIATGMVGALLLLLVVALGIGLFM
**AHP16026 CAR: SEQ ID NO: 61** 
MLLLVTSLLLCELPHPAFLLIPQVQLVESGGGLVQPGGSLRLSCAVLGSIEQINDINYYRWYRQPPGKQRDLVAS
INNSGELIYADSVKGRFFISRDKYTVYLEMRSLKAEDTAVYYCNAQDRGEYWGRGTQVTVSSESKYGPPCPPC
PFWVLVVVGGVLACYSLLVTVAFIIFWVKRGRKKLLYIFKQPFMRPVQTTQEEDGCSCRFPEEEEGGCELRVKF
SRSADAPAYQQGQNQLYNELNLGRREEYDVLDKRRGRDPEMGGKPRRKNPQEGLYNELQKDKMAEAYSEI
GMKGERRRGKGHDGLYQGLSTATKDTYDALHMQALPPRLEGGGEGRGSLLTCGDVEENPGPRMLLLVTSLL
LCELPHPAFLLIPRKVCNGIGIGEFKDSLSINATNIKHFKNCTSISGDLHILPVAFRGDSFTHTPPLDPQELDILKTV
KEITGFLLIQAWPENRTDLHAFENLEIIRGRTKQHGQFSLAVVSLNITSLGLRSLKEISDGDVIISGNKNLCYANTI
NWKKLFGTSGQKTKIISNRGENSCKATGQVCHALCSPEGCWGPEPRDCVSCRNVSRGRECVDKCNLLEGEPR
EFVENSECIQCHPECLPQAMNITCTGRGPDNCIQCAHYIDGPHCVKTCPAGVMGENNTLVWKYADAGHVC
HLCHPNCTYGCTGPGLEGCPTNGPKIPSIATGMVGALLLLLVVALGIGLFM
**AHP15547 CAR: SEQ ID NO: 62** 
MLLLVTSLLLCELPHPAFLLIPAVQLVDSGGGSVQPGESLTLSCTGSARFLSNYGMGWYRQFPGKMREMVAY
ETNRGNKRYAESVKGRFRIARDAAKTTVYLEMNSLKPEDTAVYYCNAESSGWGRRNYWGQGTQVTVSSESK
YGPPCPPCPFWVLVVVGGVLACYSLLVTVAFIIFWVKRGRKKLLYIFKQPFMRPVQTTQEEDGCSCRFPEEEEG
GCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPRLEGGGEGRGSLLTCGDVEENPGPR
MLLLVTSLLLCELPHPAFLLIPRKVCNGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVSRGRECVDK
CNLLEGEPREFVENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
**AHP15776 CAR: SEQ ID NO: 63** 
MLLLVTSLLLCELPHPAFLLIPQVQLVESGGGLVQPGGSLTLSCAASQSISSIYLLGWYRQAPGKERESVARITTS
GTTTYIADSVKGRFTLSRDNAKNTMYLQMNSLKPEDTAVYYCNVPFLVATARGNAYWGQGTQVTVSSESKY
 
GPPCPPCPFWVLVVVGGVLACYSLLVTVAFIIFWVKRGRKKLLYIFKQPFMRPVQTTQEEDGCSCRFPEEEEG
GCELRVKFSRSADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPRLEGGGEGRGSLLTCGDVEENPGPR
MLLLVTSLLLCELPHPAFLLIPRKVCNGIGIGEFKDSLSINATNIKHFKNCTSISGDLHILPVAFRGDSFTHTPPLDP
QELDILKTVKEITGFLLIQAWPENRTDLHAFENLEIIRGRTKQHGQFSLAVVSLNITSLGLRSLKEISDGDVIISGN
KNLCYANTINWKKLFGTSGQKTKIISNRGENSCKATGQVCHALCSPEGCWGPEPRDCVSCRNVSRGRECVDK
CNLLEGEPREFVENSECIQCHPECLPQAMNITCTGRGPDNCIQCAHYIDGPHCVKTCPAGVMGENNTLVWK
YADAGHVCHLCHPNCTYGCTGPGLEGCPTNGPKIPSIATGMVGALLLLLVVALGIGLFM
**R12 CAR: SEQ ID NO: 64** 
MLLLVTSLLLCELPHPAFLLIPQEQLVESGGRLVTPGGSLTLSCKASGFDFSAYYMSWVRQAPGKGLEWIATIYP
SSGKTYYATWVNGRFTISSDNAQNTVDLQMNSLTAADRATYFCARDSYADDGALFNIWGPGTLVTISSGGG
GSGGGGSGGGGSELVLTQSPSVSAALGSPAKITCTLSSAHKTDTIDWYQQLQGEAPRYLMQVQSDGSYTKRP
GVPDRFSGSSSGADRYLIIPSVQADDEADYYCGADYIGGYVFGGGTQLTVTGESKYGPPCPPCPFWVLVVVG
GVLACYSLLVTVAFIIFWVKRGRKKLLYIFKQPFMRPVQTTQEEDGCSCRFPEEEEGGCELRVKFSRSADAPAY
QQGQNQLYNELNLGRREEYDVLDKRRGRDPEMGGKPRRKNPQEGLYNELQKDKMAEAYSEIGMKGERRR
GKGHDGLYQGLSTATKDTYDALHMQALPPRLEGGGEGRGSLLTCGDVEENPGPRMLLLVTSLLLCELPHPAFL
LIPRKVCNGIGIGEFKDSLSINATNIKHFKNCTSISGDLHILPVAFRGDSFTHTPPLDPQELDILKTVKEITGFLLIQA
WPENRTDLHAFENLEIIRGRTKQHGQFSLAVVSLNITSLGLRSLKEISDGDVIISGNKNLCYANTINWKKLFGTS
GQKTKIISNRGENSCKATGQVCHALCSPEGCWGPEPRDCVSCRNVSRGRECVDKCNLLEGEPREFVENSECIQ
CHPECLPQAMNITCTGRGPDNCIQCAHYIDGPHCVKTCPAGVMGENNTLVWKYADAGHVCHLCHPNCTYG
CTGPGLEGCPTNGPKIPSIATGMVGALLLLLVVALGIGLFM
**The amino acid sequence of the heavy chain variable region of the single domain antibody AHF22013: SEQ ID NO: 65** 
EVQLVESGGGLVQPGGSLRLSCAASGRSFSSFQMGWFRQAPGKEREFVAASGWSGGPTYYWDSVIGRFTIS
TDNSKNTLYLQMNSLRAEDTAVYYCYEDRVLSGRHVRYWGQGTLVTVSS
**The amino acid sequence of the CDR1 region of AHF22013: SEQ ID NO: 66**
   GRSFSSFQ
**The amino acid sequence of the CDR2 region of AHF22013: SEQ ID NO: 3**
   SGWSGG PT
**The amino acid sequence of the CDR3 region of AHF22013: SEQ ID NO: 67**
   YEDRVLSGRHVRY
**The amino acid sequence of the heavy chain variable region of the single domain antibody AHF22016: SEQ ID NO: 68** 
EVQLVESGGGLVQPGGSLRLSCAASGRSFSSFQMGWFRQAPGKEREFVAASGWSGGPTYYWDSWKGRFTI
STDNSKNTLYLQMNSLRAEDTAVYYCYEDKVLSGRRVRYWGQGTLVTVSS
**The amino acid sequence of the CDR1 region of AHF22016: SEQ ID NO: 66**
   GRSFSSFQ
**The amino acid sequence of the CDR2 region of AHF22016: SEQ ID NO: 3**
   SGWSGG PT
**The amino acid sequence of the CDR3 region of AHF22016: SEQ ID NO: 69**
   YEDKVLSGRRVRY
**The amino acid sequence of the heavy chain variable region of the single domain antibody AHF22018: SEQ ID NO: 70** 
EVQLVESGGGLVQPGGSLRLSCAASGRSFSSFQMGWFRQAPGKEREFVAASGWSGGPTYYWDSWKGRFTI
STDNSKNTLYLQMNSLRAEDTAVYYCYEDRVLSGRWVRYWGQGTLVTVSS
**The amino acid sequence of the CDR1 region of AHF22018: SEQ ID NO: 66**
   GRSFSSFQ
**The amino acid sequence of the CDR2 region of AHF22018: SEQ ID NO: 3**
   SGWSGG PT
**The amino acid sequence of the CDR3 region of AHF22018: SEQ ID NO: 71**
   YEDRVLSGRWVRY
**The amino acid sequence of the heavy chain variable region of the single domain antibody AHP15485-VHHH4-Variant1: SEQ ID NO: 72** 
EVQLVESGGGLVQPGGSLRLSCAASGRSFSNFQMGWFRQAPGKEREFVAASGWSGGPTYYWDSVIGRFTIS
TDNSKNTLYLQMNSLRAEDTAVYYCYEDRVLSGRPVRYWGQGTLVTVSS
**The amino acid sequence of the CDR1 region of AHP15485-VHHH4-Variant1: SEQ ID NO: 2**
   GRSFSNFQ
**The amino acid sequence of the CDR2 region of AHP15485-VHHH4-Variant1: SEQ ID NO: 3**
   SGWSGG PT
**The amino acid sequence of the CDR3 region of AHP15485-VHHH4-Variant1: SEQ ID NO: 4**
   YEDRVLSGRPVRY
**The amino acid sequence of the heavy chain variable region of the single domain antibody AHP15485-VHHH4-Variant2: SEQ ID NO: 73** 
EVQLVESGGGLVQPGGSLRLSCAASGRSFSSFQMGWFRQAPGKEREFVAASGWSGGPTYYWDSVIGRFTIS
TDNSKNTLYLQMNSLRAEDTAVYYCYEDRVLSGRPVRYWGQGTLVTVSS
**The amino acid sequence of the CDR1 region of AHP15485-VHHH4-Variant2: SEQ ID NO: 66**
   GRSFSSFQ
**The amino acid sequence of the CDR2 region of AHP15485-VHHH4-Variant2: SEQ ID NO: 3**
   SGWSGG PT
**The amino acid sequence of the CDR3 region of AHP15485-VHHH4-Variant2: SEQ ID NO: 4**
   YEDRVLSGRPVRY
**The amino acid sequence of the heavy chain variable region of the single domain antibody AHP15662-VHH4-Variant1: SEQ ID NO: 74** 
EVQLVESGGGLVQPGGSLRLSCAASYYISRFYAVGWYRQAPGKQRELVARISSGGWTYYADSVKGRFTISIDN
SKNTLYLQMNSLRAEDTAVYYCNARNLWGQGTLVTVSS
**The amino acid sequence of the CDR1 region of AHP15662-VHH4-Variant1: SEQ ID NO: 75**
   YYISRFYA
**The amino acid sequence of the CDR2 region of AHP15662-VHH4-Variant1: SEQ ID NO: 76**
   ISSGGWT
**The amino acid sequence of the CDR3 region of AHP15662-VHH4-Variant1: SEQ ID NO: 19**
   NARNL
**The amino acid sequence of the heavy chain variable region of the single domain antibody AHP15662-VHH4-Variant2: SEQ ID NO: 77** 
EVQLVESGGGLVQPGGSLRLSCAASYFISRFYAVGWYRQAPGKQRELVARISSGGWTYYADSVKGRFTISIDN
SKNTLYLQMNSLRAEDTAVYYCNARNLWGQGTLVTVSS
**The amino acid sequence of the CDR1 region of AHP15662-VHH4-Variant2: SEQ ID NO: 78**
   YFISRFYA
**The amino acid sequence of the CDR2 region of AHP15662-VHH4-Variant2: SEQ ID NO: 76**
   ISSGGWT
**The amino acid sequence of the CDR3 region of AHP15662-VHH4-Variant2: SEQ ID NO: 19**
   NARNL
**The amino acid sequence of the heavy chain variable region of the single domain antibody AHP15662-VHH4-Variant3: SEQ ID NO: 79** 
EVQLVESGGGLVQPGGSLRLSCAASHYISRFYAVGWYRQAPGKQRELVARISSGGWTYYADSVKGRFTISIDN
SKNTLYLQMNSLRAEDTAVYYCNARNLWGQGTLVTVSS
**The amino acid sequence of the CDR1 region of AHP15662-VHH4-Variant3: SEQ ID NO: 80**
   HYISRFYA
**The amino acid sequence of the CDR2 region of AHP15662-VHH4-Variant3: SEQ ID NO: 76**
   ISSGGWT
**The amino acid sequence of the CDR3 region of AHP15662-VHH4-Variant3: SEQ ID NO: 19**
   NARNL
**The amino acid sequence of the heavy chain variable region of the single domain antibody AHF21711: SEQ ID NO: 81** 
EVQLVESGGGLVQPGGSLRLSCAASHSISRFYAIGWYRQAPGKQRELVARISSMGRTYYADSVKGRFTISIDNS
KNTLYLQMNSLRAEDTAVYYCNARNLWGQGTLVTVSS
**The amino acid sequence of the CDR1 region of AHF21711: SEQ ID NO: 82**
   HSISRFYA
**The amino acid sequence of the CDR2 region of AHF21711: SEQ ID NO: 83**
   ISSMGRT
**The amino acid sequence of the CDR3 region of AHF21711: SEQ ID NO: 19**
   NARNL
**The amino acid sequence of the heavy chain variable region of the single domain antibody AHF21719: SEQ ID NO: 84** 
EVQLVESGGGLVQPGGSLRLSCAASGYISRFYAMGWYRQAPGKQRELVARISSGGWTYYADSVKGRFTISID
NSKNTLYLQMNSLRAEDTAVYYCNARNLWGQGTLVTVSS
**The amino acid sequence of the CDR1 region of AHF21719: SEQ ID NO: 85**
   GYISRFYA
**The amino acid sequence of the CDR2 region of AHF21719: SEQ ID NO: 76**
   ISSGGWT
**The amino acid sequence of the CDR3 region of AHF21719: SEQ ID NO: 19**
   NARNL
**The amino acid sequence of the heavy chain variable region of the single domain antibody AHF21720: SEQ ID NO: 86** 
EVQLVESGGGLVQPGGSLRLSCAASGYISRFYAMGWYRQAPGKQRELVARISSLGWTYYADSVKGRFTISIDN
SKNTLYLQMNSLRAEDTAVYYCNARNLWGQGTLVTVSS
**The amino acid sequence of the CDR1 region of AHF21720: SEQ ID NO: 85**
   GYISRFYA
**The amino acid sequence of the CDR2 region of AHF21720: SEQ ID NO: 87**
   ISSLGWT
**The amino acid sequence of the CDR3 region of AHF21720: SEQ ID NO: 19**
   NARNL
**The amino acid sequence of the heavy chain variable region of the single domain antibody AHF21721: SEQ ID NO: 88** 
EVQLVESGGGLVQPGGSLRLSCAASGFISRFYAMGWYRQAPGKQRELVARISSMGWTYYADSVKGRFTISID
NSKNTLYLQMNSLRAEDTAVYYCNARNLWGQGTLVTVSS
**The amino acid sequence of the CDR1 region of AHF21721: SEQ ID NO: 89**
   GFISRFYA
**The amino acid sequence of the CDR2 region of AHF21721: SEQ ID NO: 90**
   ISSMGWT
**The amino acid sequence of the CDR3 region of AHF21721: SEQ ID NO: 19**
   NARNL

## Claims

1. An antibody or antigen-binding fragment thereof targeting a ROR1 protein, wherein the antibody comprises a heavy chain variable region comprising HCDR1, HCDR2, and HCDR3 selected from one of the following combinations:
(1) an amino acid sequence of HCDR1 as set forth in SEQ ID NO: 2;
an amino acid sequence of HCDR2 as set forth in SEQ ID NO: 3; and
an amino acid sequence of HCDR3 as set forth in SEQ ID NO: 4;
(2) an amino acid sequence of HCDR1 as set forth in SEQ ID NO: 7;
an amino acid sequence of HCDR2 as set forth in SEQ ID NO: 8; and
an amino acid sequence of HCDR3 as set forth in SEQ ID NO: 9;
(3) an amino acid sequence of HCDR1 as set forth in SEQ ID NO: 12;
an amino acid sequence of HCDR2 as set forth in SEQ ID NO: 13; and
an amino acid sequence of HCDR3 as set forth in SEQ ID NO: 14;
(4) an amino acid sequence of HCDR1 as set forth in SEQ ID NO: 17;
an amino acid sequence of HCDR2 as set forth in SEQ ID NO: 18; and
an amino acid sequence of HCDR3 as set forth in SEQ ID NO: 19;
(5) an amino acid sequence of HCDR1 as set forth in SEQ ID NO: 22;
an amino acid sequence of HCDR2 as set forth in SEQ ID NO: 23; and
an amino acid sequence of HCDR3 as set forth in SEQ ID NO: 24;
(6) an amino acid sequence of HCDR1 as set forth in SEQ ID NO: 27;
an amino acid sequence of HCDR2 as set forth in SEQ ID NO: 28; and
an amino acid sequence of HCDR3 as set forth in SEQ ID NO: 29;
(7) an amino acid sequence of HCDR1 as set forth in SEQ ID NO: 32;
an amino acid sequence of HCDR2 as set forth in SEQ ID NO: 33; and
an amino acid sequence of HCDR3 as set forth in SEQ ID NO: 34;
(8) an amino acid sequence of HCDR1 as set forth in SEQ ID NO: 37;
an amino acid sequence of HCDR2 as set forth in SEQ ID NO: 38; and
an amino acid sequence of HCDR3 as set forth in SEQ ID NO: 39;
(9) an amino acid sequence of HCDR1 as set forth in SEQ ID NO: 66;
an amino acid sequence of HCDR2 as set forth in SEQ ID NO: 3; and
an amino acid sequence of HCDR3 as set forth in SEQ ID NO: 67;
(10) an amino acid sequence of HCDR1 as set forth in SEQ ID NO: 66;
an amino acid sequence of HCDR2 as set forth in SEQ ID NO: 3; and
an amino acid sequence of HCDR3 as set forth in SEQ ID NO: 71;
(11) an amino acid sequence of HCDR1 as set forth in SEQ ID NO: 66;
an amino acid sequence of HCDR2 as set forth in SEQ ID NO: 3; and
an amino acid sequence of HCDR3 as set forth in SEQ ID NO: 4;
(12) an amino acid sequence of HCDR1 as set forth in SEQ ID NO: 75;
an amino acid sequence of HCDR2 as set forth in SEQ ID NO: 76; and
an amino acid sequence of HCDR3 as set forth in SEQ ID NO: 19;
(13) an amino acid sequence of HCDR1 as set forth in SEQ ID NO: 78;
an amino acid sequence of HCDR2 as set forth in SEQ ID NO: 76; and
an amino acid sequence of HCDR3 as set forth in SEQ ID NO: 19;
(14) an amino acid sequence of HCDR1 as set forth in SEQ ID NO: 80;
an amino acid sequence of HCDR2 as set forth in SEQ ID NO: 76; and
an amino acid sequence of HCDR3 as set forth in SEQ ID NO: 19;
(15) an amino acid sequence of HCDR1 as set forth in SEQ ID NO: 82;
an amino acid sequence of HCDR2 as set forth in SEQ ID NO: 83; and
an amino acid sequence of HCDR3 as set forth in SEQ ID NO: 19;
(16) an amino acid sequence of HCDR1 as set forth in SEQ ID NO: 85;
an amino acid sequence of HCDR2 as set forth in SEQ ID NO: 76; and
an amino acid sequence of HCDR3 as set forth in SEQ ID NO: 19;
(17) an amino acid sequence of HCDR1 as set forth in SEQ ID NO: 85;
an amino acid sequence of HCDR2 as set forth in SEQ ID NO: 87; and
an amino acid sequence of HCDR3 as set forth in SEQ ID NO: 19; and
(18) an amino acid sequence of HCDR1 as set forth in SEQ ID NO: 89;
an amino acid sequence of HCDR2 as set forth in SEQ ID NO: 90; and
an amino acid sequence of HCDR3 as set forth in SEQ ID NO: 19;
or
the antibody is a variant of an antibody defined by the amino acid sequences of HCDR1, HCDR2, and HCDR3 in any one of (1) to (18), wherein the variant comprises at least 1 and no more than 10, 9, 8, 7, 6, 5, 4, 3, or 2 amino acid changes in total in the sequences of HCDR1, HCDR2, and HCDR3, in comparison to the antibody defined in any one of (1) to (18).

2. The antibody or antigen-binding fragment thereof of claim 1, wherein an amino acid sequence of the heavy chain variable region is selected from any one of:
(1) a sequence as set forth in SEQ ID NO: 1 or a heavy chain variable region sequence having at least 90% sequence identity thereto;
(2) a sequence as set forth in SEQ ID NO: 6 or a heavy chain variable region sequence having at least 90% sequence identity thereto;
(3) a sequence as set forth in SEQ ID NO: 11 or a heavy chain variable region sequence having at least 90% sequence identity thereto;
(4) a sequence as set forth in SEQ ID NO: 16 or a heavy chain variable region sequence having at least 90% sequence identity thereto;
(5) a sequence as set forth in SEQ ID NO: 21 or a heavy chain variable region sequence having at least 90% sequence identity thereto;
(6) a sequence as set forth in SEQ ID NO: 26 or a heavy chain variable region sequence having at least 90% sequence identity thereto;
(7) a sequence as set forth in SEQ ID NO: 31 or a heavy chain variable region sequence having at least 90% sequence identity thereto;
(8) a sequence as set forth in SEQ ID NO: 36 or a heavy chain variable region sequence having at least 90% sequence identity thereto;
(9) a sequence as set forth in SEQ ID NO: 65 or a heavy chain variable region sequence having at least 90% sequence identity thereto;
(10) a sequence as set forth in SEQ ID NO: 70 or a heavy chain variable region sequence having at least 90% sequence identity thereto;
(11) a sequence as set forth in SEQ ID NO: 81 or a heavy chain variable region sequence having at least 90% sequence identity thereto;
(12) a sequence as set forth in SEQ ID NO: 84 or a heavy chain variable region sequence having at least 90% sequence identity thereto;
(13) a sequence as set forth in SEQ ID NO: 86 or a heavy chain variable region sequence having at least 90% sequence identity thereto; and
(14) a sequence as set forth in SEQ ID NO: 88 or a heavy chain variable region sequence having at least 90% sequence identity thereto.

3. The antibody or antigen-binding fragment thereof of claim 1 or 2, wherein the antibody is a single domain antibody.

4. The antibody or antigen-binding fragment thereof of any one of claims 1-3, wherein the antibody is a humanized antibody.

5. The antibody or antigen-binding fragment thereof of any one of claims 1-4, wherein an amino acid sequence of a heavy chain variable region of the humanized antibody is selected from any one of:
(1) a sequence as set forth in SEQ ID NO: 41 or a heavy chain variable region sequence having at least 90% sequence identity thereto;
(2) a sequence as set forth in SEQ ID NO: 43 or a heavy chain variable region sequence having at least 90% sequence identity thereto;
(3) a sequence as set forth in SEQ ID NO: 45 or a heavy chain variable region sequence having at least 90% sequence identity thereto;
(4) a sequence as set forth in SEQ ID NO: 72 or a heavy chain variable region sequence having at least 90% sequence identity thereto;
(5) a sequence as set forth in SEQ ID NO: 73 or a heavy chain variable region sequence having at least 90% sequence identity thereto;
(6) a sequence as set forth in SEQ ID NO: 74 or a heavy chain variable region sequence having at least 90% sequence identity thereto;
(7) a sequence as set forth in SEQ ID NO: 77 or a heavy chain variable region sequence having at least 90% sequence identity thereto; and
(8) a sequence as set forth in SEQ ID NO: 79 or a heavy chain variable region sequence having at least 90% sequence identity thereto.

6. The antibody or antigen-binding fragment thereof of any one of claims 1-5, wherein the antibody targets the Frizzled domain or the Kringle domain of the ROR1 protein.

7. The antibody or antigen-binding fragment thereof of any one of claims 1-6, wherein the antibody has an EC₅₀ value of no higher than 0.1 µg/mL for the binding to the ROR1 protein, or the Frizzled domain or Kringle domain thereof as determined by ELISA.

8. The antibody or antigen-binding fragment thereof of any one of claims 1-7, wherein the antibody has a KD value of no higher than 10⁻⁶ M, preferably no higher than 10⁻⁷ M, more preferably no higher than 10⁻⁸ M for the binding to the ROR1 protein, or the Frizzled domain or Kringle domain thereof as determined by surface plasmon resonance technology.

9. The antibody or antigen-binding fragment thereof of any one of claims 1-8, wherein the antibody also comprises an Fc fragment; preferably, the Fc fragment is derived from a human IgG, such as IgG1.

10. A fusion protein comprising at least one antigen-binding functional portion, wherein the antigen-binding functional portion comprises the antibody or antigen-binding fragment thereof of any one of claims 1-9.

11. The fusion protein of claim 10, comprising at least two antigen-binding functional portions targeting the same or different antigen epitopes, respectively.

12. The fusion protein of claim 10 or 11, wherein:
one of the two antigen-binding functional portions targets the Frizzled domain of the ROR1 protein, and the other of the two antigen-binding functional portions targets other portions on the ROR1 protein other than the Frizzled domain;
one of the two antigen-binding functional portions targets the Kringle domain of ROR1, and the other of the two antigen-binding functional portions targets other portions on the ROR1 protein other than the Kringle domain; or
one of the two antigen-binding functional portions targets the Kringle domain of ROR1, and the other of the two antigen-binding functional portions targets the Frizzled domain of ROR1.

13. The fusion protein of any one of claims 10-12, wherein the antigen-binding functional portions are linked by a peptide linker molecule.

14. A chimeric antigen receptor targeting a ROR1 protein, wherein the chimeric antigen receptor comprises an extracellular antigen binding domain comprising the antibody or antigen-binding fragment of any one of claims 1-9, or the fusion protein of any one of claims 10-13.

15. The chimeric antigen receptor of claim 14, comprising an amino acid sequence as set forth in any one of SEQ ID NOs: 56-63.

16. A nucleic acid molecule encoding the antibody or antigen-binding fragment thereof of any one of claims 1-9, the fusion protein of any one of claims 10-13, or the chimeric antigen receptor of claim 14 or 15.

17. The nucleic acid molecule of claim 16, comprising a nucleotide sequence as set forth in any one of SEQ ID NOs: 5, 10, 15, 20, 25, 30, 35, 40, 42, 44, and 46.

18. An expression vector comprising the nucleic acid molecule of claim 16 or 17.

19. A host cell comprising the expression vector of claim 18 or expressing the antibody or antigen-binding fragment thereof of any one of claims 1-9, the fusion protein of any one of claims 10-13, or the chimeric antigen receptor of claim 14 or 15.

20. The host cell of claim 19, wherein the host cell is an immune effector cell and expresses the chimeric antigen receptor of either claim 14 or 15.

21. The host cell of claim 20, wherein the immune effector cell is a T cell or an NK cell.

22. A pharmaceutical composition comprising:
1) the antibody or antigen-binding fragment thereof of any one of claims 1-9, the fusion protein of any one of claims 10-13, the nucleic acid molecule of claim 16 or 17, or the host cell of any one of claims 19-21; and
2) a pharmaceutically acceptable carrier.

23. A method for treating a disease comprising administering to a subject in need thereof an effective amount of the antibody or antigen-binding fragment thereof of any one of claims 1-9, the fusion protein of any one of claims 10-13, the nucleic acid molecule of claim 16 or 17, the host cell of any one of claims 19-21, or the pharmaceutical composition of claim 22.

24. The method of claim 23, wherein the disease is a tumor expressing the ROR1 protein.

25. The method of claim 24, wherein the tumor is selected from chronic lymphocytic leukemia, mantle cell lymphoma, and ovarian cancer.

26. A kit for detecting a ROR1 protein in a sample, wherein the kit comprises the antibody or antigen-binding fragment thereof of any one of claims 1-9, or the fusion protein of any one of claims 10-13.

27. Use of the antibody or antigen-binding fragment thereof of any one of claims 1-9, the fusion protein of any one of claims 10-13, the nucleic acid molecule of claim 16 or 17, or the host cell of any one of claims 19-21, in the manufacture of a medicament for the treatment of a tumor.

28. The use of claim 27, wherein the disease is a tumor expressing the ROR1 protein.

29. The use of claim 28, wherein the tumor is selected from chronic lymphocytic leukemia, mantle cell lymphoma, and ovarian cancer.
